(19) Europäisches Patentamt
European Patent Office
Office européen des brevets

(11) EP 3 486 195 A1

(12) **EUROPEAN PATENT APPLICATION**
published in accordance with Art. 153(4) EPC

(43) Date of publication:
**22.05.2019 Bulletin 2019/21**

(51) Int Cl.:
**B65D 85/672** (2006.01)  **C09J 7/24** (2018.01)

(21) Application number: **17827603.6**

(22) Date of filing: **11.07.2017**

(86) International application number:
**PCT/JP2017/025199**

(87) International publication number:
**WO 2018/012476 (18.01.2018 Gazette 2018/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **12.07.2016 JP 2016137602**
**28.04.2017 JP 2017089940**
**20.06.2017 JP 2017120231**

(71) Applicant: **Asahi Kasei Kabushiki Kaisha Tokyo 1000006 (JP)**

(72) Inventors:
• **KISHI, Mio**
**Tokyo 101-8101 (JP)**
• **IRIYA, Masaru**
**Tokyo 101-8101 (JP)**
• **ISHIDA, Masayuki**
**Tokyo 101-8101 (JP)**

(74) Representative: **D Young & Co LLP**
**120 Holborn**
**London EC1N 2DY (GB)**

(54) **BOX FOR HOUSING PRESSURE-SENSITIVE - ADHESIVE FILM THEREIN, DEVICE INCLUDING PRESSURE-SENSITIVE-ADHESIVE FILM HOUSED THEREIN, AND INFECTION-PREVENTING FILM**

(57)    Provided is an adhesive film storage box including a main body and a cover. The main body has at least a front plate, a bottom plate, a back plate, and side plates, and has an open upper surface. The cover has at least a cover plate and a covering piece. The cover plate is connected to an upper edge of the back plate of the main body so as to oppose the upper surface of the main body. The covering piece is connected to a front edge of the cover plate so as to oppose a front surface of the main body. A cutting blade capable of cutting an adhesive film is provided on a lower edge of the covering piece. The height of the front plate is set to be lower than the height of the back plate and the side plates and to be between 50% and 90% of an outer diameter of a core tube of an adhesive film roll.

Fig. 2

**Description**

**Technical Field**

The present application is based on Japanese Patent Application No. 2016-137602, filed on July 12, 2016, Japanese Patent Application No. 2017-089940, filed on April 28, 2017, and Japanese Patent Application No. 2017-120231, filed on June 20, 2017, the content of these applications being incorporated herein.

The present application relates to an adhesive film storage box for storing a roll of wound highly adhesive film such as an infection prevention film, and an adhesive film storage device in which the roll is stored in the adhesive film storage box.

**Background Art**

In a medical setting, for example, a doctor may create a medical chart or the like by inputting patient information into a personal computer while examining and treating the patient. At this time, the doctor may touch the keyboard of the personal computer with a hand that has touched the patient. When bodily fluid or the like from the patient sticks to the keyboard, the risk of infection or the like arises among healthcare workers such as doctors and nurses, which is undesirable.

Citation List

Patent Document

Patent Document 1: Patent Publication JP-A-2015-83484

**Summary**

Technical Problem

Therefore, for example, a highly adhesive disposable infection prevention film may be adhered to the surface of the keyboard of the personal computer, and after using the keyboard, the film may be peeled off and discarded.

However, a storage container for an adhesive film such as the infection prevention film described above, which stores the adhesive film such that the adhesive film can be cut to a desired length during use, thereby facilitating use of the adhesive film by healthcare workers, is yet to be developed.

A wrapping film storage box that stores a roll of wrapping film formed by winding up the wrapping film is available in the prior art (see Patent Document 1), but when this wrapping film storage box is used to store a highly adhesive film such as that described above, the adhesive film may adhere to a front plate of the box when the adhesive film is pulled out of the box, for example, making it impossible to pull out the adhesive film.

Further, when the aforesaid wrapping film storage box is used to store a highly adhesive film such as that described above, the adhesive film has to be pulled with great force in order to pull the adhesive film out of the box, for example, and as a result, the roll of wound adhesive film moves violently inside the box. If, at this time, a width direction end of the adhesive film wound on the roll or the end of the adhesive film that has just been pulled from the roll contacts a side wall face of the box, dust may adhere to the adhesive film. When dust adheres to the adhesive film, the adhesiveness of the adhesive film decreases, which is undesirable. This is also undesirable in terms of hygiene.

The present application has been designed in consideration of the points described above, and an object thereof is to provide an adhesive film storage box and an adhesive film storage device that can store an adhesive film so that the adhesive film can be pulled out easily during use.

Another object of the present application is to provide an adhesive film storage box and an adhesive film storage device that exhibit superior qualities in terms of use and adhesive film storage, and with which the adhesive film can be pulled out for use so that no dust adheres thereto.

Solution to Problem

As a result of much committed research, the present inventor found that the problems described above are solved by making the height of a front plate of an adhesive film storage box lower than the height of a back plate and side plates, and thus arrived at the present invention. Further, as a result of much committed research, the present inventor found that the problems described above are solved by providing extension pieces on respective side walls of the adhesive film storage box, and thus arrived at the present invention. More specifically, an aspect of the present invention includes the following configurations.
(1) An adhesive film storage box for storing a roll of wound adhesive film having an adhesive property, the adhesive film storage box including a main body having at least a front plate, a bottom plate, a back plate, and side plates, and having an open upper surface, and a cover capable of opening and closing an opening in the upper surface of the main body, wherein the cover includes at least a cover plate connected to an upper edge of the back plate of the main body so as to oppose the upper surface of the main body, and a covering piece connected to a front edge of the cover plate so as to oppose a front surface of the main body, a cutting portion capable of cutting the adhesive film is provided on a lower edge of the covering piece, and a height of the front plate is set to be lower than a height of the back plate and the side plates and to be between 50% and 90% of an outer diameter of a core tube of the roll.
(2) The adhesive film storage box described in (1), wherein the adhesive film has an adhesive force (a 180-degree peel strength compliant with JIS 0237) between 0.01 and 1.5 N / 25 mm.
(3) The adhesive film storage box described in (1) or (2), wherein a peel strength of the adhesive film relative to the front plate is between 0.5 and 5 N / 220 mm.
(4) The adhesive film storage box described in any of (1) to (3), wherein a pull-out resistance for pulling the adhesive film from the core tube is between 30 and 300 cN / 200 mm width.
(5) The adhesive film storage box described in any of (1) to (4), wherein the adhesive film storage box stores the roll which is formed by winding the adhesive film such that when a surface area of the core tube projected from above is set at 100%, 97% to 100% of the surface area of the core tube is covered by the adhesive film.
(6) The adhesive film storage box described in any of (1) to (5), wherein the main body has an extension piece that extends into the main body from an upper portion of each side wall on each longitudinal direction side of the main body, and the extension piece is configured to be capable of pushing the roll inside the main body toward a center side of the main body.
(7) The adhesive film storage box described in (6), wherein the extension piece is capable of extending into the core tube of the roll inside the main body.
(8) The adhesive film storage box described in (7), wherein the extension piece is narrower in width at a tip end than at a base end.
(9) The adhesive film storage box described in (7) or (8), wherein the extension piece has a first part connected to an upper end of the side wall and a second part connected to the first part, the first part gradually decreases in width toward a tip end side, and the second part has an unvarying width.
(10) The adhesive film storage box described in any of (6) to (9), wherein the side wall has at least a first side plate facing an interior of the main body and a second side plate positioned on an outside of the first side plate, the first side plate includes a folded-in portion that enters the core tube of the roll in the main body, the folded-in portion is formed by folding a part surrounded by a cut line formed in the first side plate inward, and an opening in the first side plate, which is formed by folding the folded-in portion, is closed by the second side plate.
(11) An adhesive film storage device in which the roll is stored in the adhesive film storage box described in any of (1) to (10).
(12) An infection prevention film including a base material film that has a 2% strain modulus of elasticity in each of a flow direction (an MD direction) and a width direction (a TD direction) of 190 to 600 MPa, and a tensile elongation in each of the flow direction (the MD direction) and the width direction (the TD direction) of 60% to 200%.
(13) The infection prevention film described in (12), further including an adhesive layer, wherein an adhesive force of the adhesive layer (a 180-degree peel strength compliant with JIS 0237) is between 0.01 and 1.5 N / 25 mm.
(14) The infection prevention film described in (13), wherein a haze of a layer including the base material film and the adhesive layer is between 0.1 and 25.
(15) The infection prevention film described in any one of (12) to (14), wherein a density is between 0.9 and 1.9 g/cm$^3$ and a thickness of the base material film is between 5 and 50 $\mu$m.
(16) The infection prevention film described in any one of (12) to (15), wherein a heat shrinkage at 120°C is between 20% and 45%.
(17) The infection prevention film described in any one of (12) to (16), wherein a principal component of the base material film is a vinylidene chloride copolymer.
(18) The infection prevention film described in (13) or (14), wherein a separator is provided on the adhesive layer side of a layer having the base material film and the adhesive layer.
(19) An infection prevention film roll including a core tube and the infection prevention film described in any one of (12) to (18), which is wound onto the core tube.
(20) The infection prevention film roll described in (19), wherein a pull-out resistance for pulling the infection prevention film from the core tube is between 30 and 300 cN / 200 mm width.
(21) An infection prevention film storage box for storing the infection prevention film roll described in (19) or (20), including means for pulling the infection prevention film from the core tube.

(22) An infection prevention film storage box for storing the infection prevention film roll described in (19) or (20), including means for cutting the infection prevention film pulled from the core tube.

Advantageous Effects of Invention

According to the present invention, the adhesive film can be stored and pulled out easily during use, and therefore a user can use the adhesive film easily.

According to the present invention, it is possible to provide an adhesive film storage box and an adhesive film storage device that exhibit superior qualities in terms of use and adhesive film storage, and with which the adhesive film can be pulled out for use so that no dust adheres thereto.

**Brief Description of Drawings**

Fig. 1 is a perspective view showing an example of the outer appearance of an adhesive film storage device.

Fig. 2 is a perspective view showing the adhesive film storage device with an open cover.

Fig. 3 is a perspective view showing configurations of an adhesive film roll and an adhesive film storage box.

Fig. 4 is a development diagram showing a front surface side of the adhesive film storage box.

Fig. 5 is an illustrative view showing a vertical section of the adhesive film storage box in order to illustrate the height of a front plate.

Fig. 6 is a perspective view of the adhesive film storage box when an opening piece is torn off.

Fig. 7 is a top view showing a main body of the adhesive film storage box with reinforcing members attached to a rear surface of the front plate.

Fig. 8 is a development diagram showing the adhesive film storage box including the reinforcing members.

Fig. 9 is a perspective view showing the adhesive film storage device including another jump-out suppressing member.

Fig. 10 is an illustrative view showing the main body from a side face.

Fig. 11 is an illustrative view of a vertical section of the main body, showing a state in which an adhesive film roll is held by folded-in portions.

Fig. 12 is a perspective view showing an example of the outer appearance of the adhesive film storage device.

Fig. 13 is a perspective view showing the adhesive film storage device with an open cover.

Fig. 14 is a perspective view showing configurations of a roll and the adhesive film storage box.

Fig. 15 is a development diagram showing the front surface side of the adhesive film storage box.

Fig. 16 is an illustrative view showing the configuration of a side portion of the adhesive film storage box.

Fig. 17 is a sectional view of the adhesive film storage device.

Fig. 18 is a plan view of the side portion of the adhesive film storage box.

Fig. 19 is an illustrative view showing the configuration of the folded-in portion of the adhesive film storage box.

Fig. 20 is an illustrative view showing a vertical section of the adhesive film storage box in order to illustrate the height of the front plate.

Fig. 21 is a perspective view of the adhesive film storage box when the opening piece is torn off.

Fig. 22 is a development diagram showing the front surface side of the adhesive film storage box when an extension piece is provided on a back plate side piece.

Fig. 23 is a table showing measurement results of examples.

## Description of Embodiments

Embodiments of the present invention will be described specifically below. Note that the embodiments to be described below are examples used to illustrate the present invention, and the present invention is not limited to these embodiments alone. Identical elements have been allocated identical reference numerals, and duplicate description thereof has been omitted. Further, in the figures, positional relationships such as up, down, left, right, and so on are assumed to be based on the positional relationships shown in the figures, unless specified otherwise. Moreover, the dimensional ratios in the figures are not limited to the illustrated ratios.

(First Embodiment)

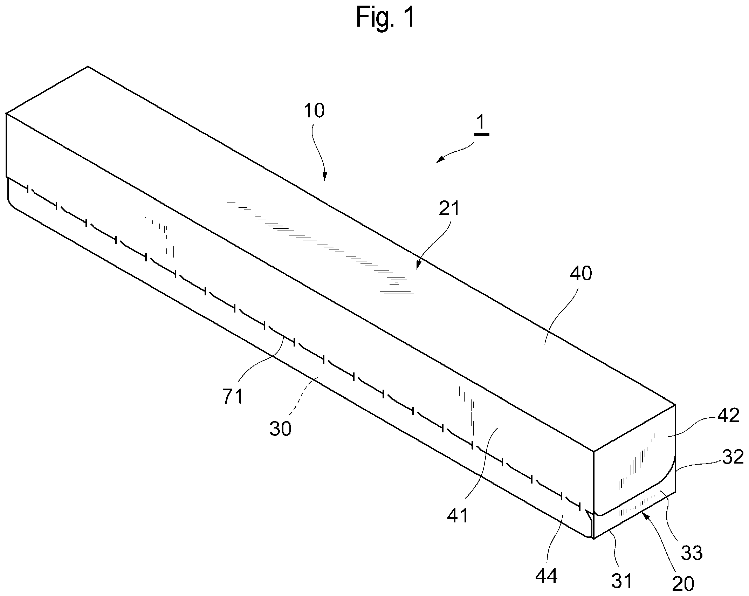

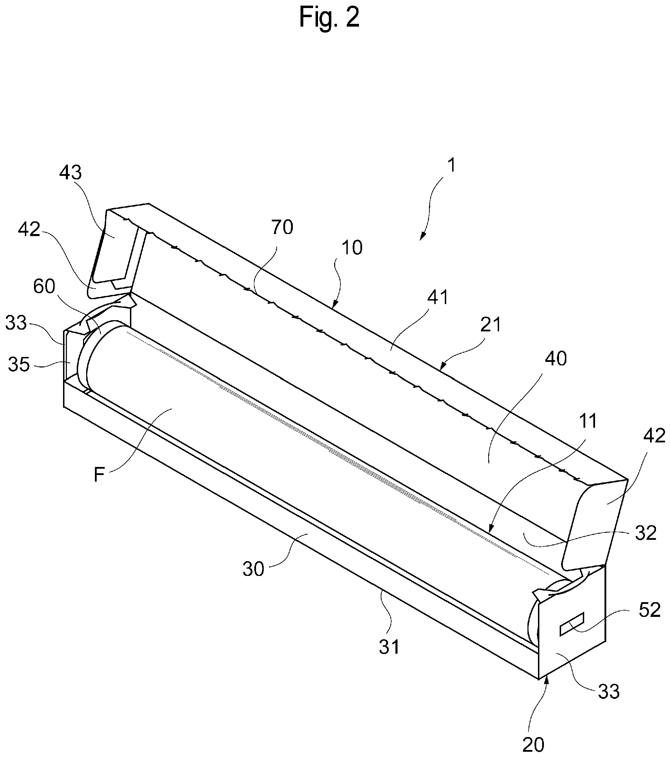

Fig. 1 is a perspective view showing an example of an adhesive film storage device 1 according to this embodiment. Fig. 2 is a perspective view showing the adhesive film storage device 1 in an open state.

The adhesive film storage device 1 includes an adhesive film storage box 10 and an adhesive film roll 11. Note that in this specification, the "adhesive film" is a film having an adhesiveness of at least 0.01 N / 25 mm.

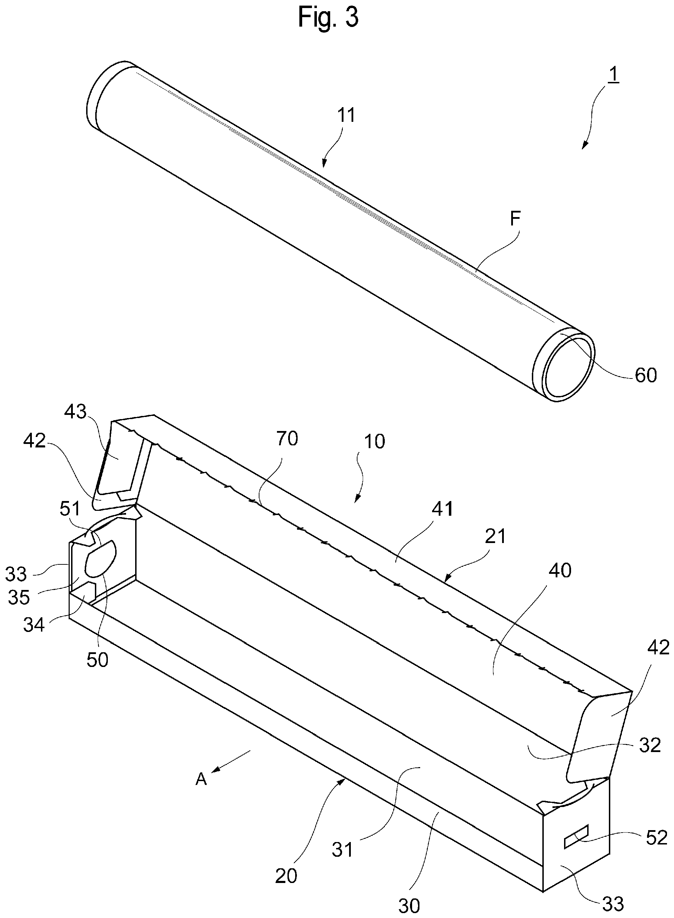

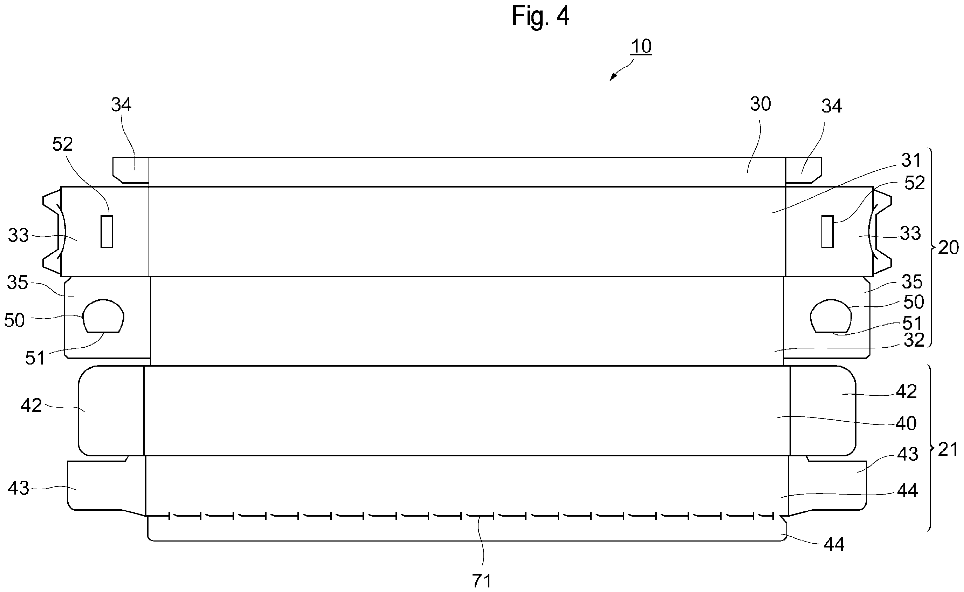

The adhesive film storage box 10 takes the overall shape of a rectangular parallelepiped, for example. As shown in Figs. 3 and 4, the adhesive film storage box 10 includes a main body 20 and a cover 21. The main body 20 includes, for example, a front plate 30, a bottom plate 31, a back plate 32, side plates 33, front plate side pieces 34 and back plate side pieces 35 serving as reinforcing members, and so on. The cover 21 includes, for example, a cover plate 40, a covering piece 41, cover plate side pieces 42, covering side pieces 43, an opening piece 44, and so on.

The main body 20 is formed in the shape of a rectangular parallelepiped with an open upper surface. The front plate 30, the bottom plate 31, the back plate 32, and the side plates 33 are each formed in a rectangular shape so as to form respective surfaces of the main body 20. The back plate side pieces 35 are overlapped onto and adhered to respective inner sides of the side plates 33.

A folded-in portion 50 serving as a jump-out suppressing member is formed in the center of each back plate side piece 35. Each folded-in portion 50 is formed in the shape of a tongue (a substantially semicircular shape) that projects downward, for example, and a fold line 51 is formed on an upper end thereof. A rectangular push-in piece 52 for pushing the folded-in portion 50 on the back plate side piece 35 from the outside in order to fold the folded-in portion 50 inward is formed on the side plate 33 overlapped onto the outside of the back plate side piece 35. During manufacture of the main body 20, the folded-in portions 50 are not folded in, but after the adhesive film roll 11 is stored in the main body 20, the push-in pieces 52 are pushed from the outside so that the folded-in portions 50 are folded into a tube forming a core tube 60, to be described below, whereby the adhesive film roll 11 is held by the folded-in portions 50. As a result, the adhesive film roll 11 can be prevented from jumping out of the main body 20.

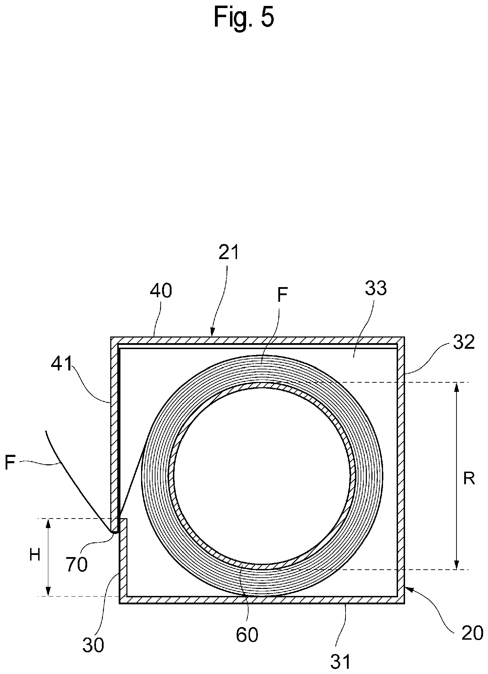

As shown in Figs. 3 to 5, the back plate 32 and the side plates 33 are formed at an approximately identical height, while the front plate 30 is formed to be lower than the back plate 32 and the side plates 33. The front plate 30 is formed in the shape of a rectangular plate having a constant height in a longitudinal direction of the adhesive film storage box 10. As shown in Fig. 5, the front plate 30 has a height H corresponding to 50% to 90%, preferably 50% to 80%, and more preferably 50% to 67% of an outer diameter R of the core tube 60 of the adhesive film roll 11, for example. Further, the height H of the front plate 30 may be set at no more than half the height of the back plate 32 and the side plates 33. Furthermore, a vertical width of a front surface opening formed in the main body 20 by the reduced height of the front plate 30 may be set to equal or exceed half the height of the back plate 32 and the side plates 33.

As shown in Fig. 3, the front plate side pieces 34 are overlapped onto and adhered to the inner sides of the back plate side pieces 35. As a result, the strength of the front plate 30 against external force acting in a frontward direction (the direction of an arrow A in Fig. 3) of the adhesive film storage box 10 is increased.

The cover plate 40 of the cover 21 extends frontward from an upper edge of the back plate 32 so as to cover an upper surface of the main body 20. The covering piece 41 extends toward a front surface (the front plate 30) side from a front edge of the cover plate 40. A tip end of the covering piece 41 overlaps the front plate 30 at least partially. A saw blade-shaped cutting blade 70 is provided on a rear side of the tip end of the covering piece 41, for example, as a cutting portion for cutting an adhesive film F pulled from the adhesive film roll 11 inside the main body 20. A metal such as tin plate, or alternatively resin or paper, may be used as the material of the cutting blade 70.

As shown in Fig. 1, before the adhesive film storage device 1 is used, the adhesive film storage box 10 is sealed by an opening piece 44 connected to the tip end of the covering piece 41 of the cover 21. The opening piece 44 is formed in the shape of a strip positioned on the front surface of the front plate 30 of the main body 20 so as to extend in the longitudinal direction of the adhesive film storage box 10. The opening piece 44 is connected tearably to the tip end of the covering piece 41 via a tear line 71 and adhered to a plurality of locations on the front surface of the front plate 30. When the adhesive film storage box 10 is to be opened, the cover 21 can be opened by tearing the opening piece 44 in the longitudinal direction of the box.

Note that cardboard, coated cardboard, corrugated cardboard, and so on may be cited as examples of base paper constituting the adhesive film storage box 10, but the present invention is not limited thereto, and any type of paper known in the industry may be selected as appropriate for use. The base paper may be formed by implementing embossing, printing, or laminating using polyethylene or the like on any of these materials.

The adhesive film roll 11 (an infection prevention film roll) is formed by winding the adhesive film F, which is a film having an adhesive property such as injection prevention film, for example, onto the core tube 60, which is cylindrical and made of paper. Infection prevention film is a film for preventing cross-infection among medical personnel in a hospital by preventing bacteria and viruses, as well as blood, vomit, and other bodily fluids, from adhering to keyboards of electronic charts, various testing equipment having operating consoles, and so on with which a plurality of medical personnel come into contact. The infection prevention film will be described in detail below. For example, the adhesive film roll 11 according to this embodiment has a longitudinal direction width of at least 100 mm, and the diameter of the core tube 60 is between approximately 25 mm and 40 mm.

The adhesive film F at least partially includes an adhesive layer, and an adhesive force (the 180-degree peel strength compliant with JIS 0237) of the adhesive layer is between 0.01 and 1.5 N / 25 mm, for example. Further, a pull-out resistance required to pull the adhesive film F from the core tube 60 is between 30 and 300 cN / 200 mm width (the pull-out resistance exerted on the 200 mm width of the adhesive film F is between 30 and 300 cN), for example.

There are no particular limitations on the adhesive used to form the adhesive layer, but an acrylic-based adhesive, a rubber-based adhesive, a silicone-based adhesive, and a urethane-based adhesive may be cited as examples, and among these adhesives, an acrylic-based adhesive or a urethane-based adhesive is preferable.

With an acrylic-based adhesive, the cohesive force can be adjusted by adding a comonomer to the principal monomer, and the hardness of the adhesive can be adjusted by adding a monomer containing a functional group so as to provide a cross-linking point. In so doing, a target adhesive force can be realized. There are no particular limitations on the principal monomer, but various types of (meta-) alkyl acrylates, such as (meta-) methyl acrylate, (meta-) ethyl acrylate, (meta-) butyl acrylate, and (meta-) 2-ethylhexyl acrylate, may be cited as examples, and these monomers may be used individually or in combination. There are no particular limitations on the comonomer, but vinyl acetate, acrylonitrile, acrylamide, styrene, methyl metacrylate, and methyl acrylate may be cited as examples. There are no particular limitations on the functional group-containing monomer, but acrylate, hydroxyethyl acrylate, acrylamide, glycidyl methacrylate, and so on may be cited as examples. Furthermore, the adhesive force can be adjusted by adding rosin, a terpene, or a tackifier such as an aliphatic or aromatic synthetic resin. A stabilizer, an antioxidant, a photostabilizer, a UV absorber, and so on may also be added to the adhesive.

There are no particular limitations on a polyurethane adhesive as long as the adhesive contains polyurethane and a curing agent. As further additives, the polyurethane adhesive may contain a coupling agent, a plasticizer, a tackifier such as a terpene-based or a rosin-based tackifier, an inorganic filler such as calcium carbide or titanium oxide, stabilizers such as an anti-oxidant and a UV absorber, and so on.

There are no particular limitations on the polyurethane as long as the polyurethane is a copolymer having a urethane bond, and polyurethane containing a hydroxyl group or the like may be cited as a favorable example. A copolymer of an isocyanate compound and a polyol may be cited as a specific example of polyurethane containing a hydroxyl group.

An aromatic diisocyanate such as methylene diphenyl diisocyanate (MDI), toluene diisocyanate (TDI), or xylylene diisocyanate (XDI), an aliphatic diisocyanate such as hexamethylene diisocyanate (HDI), and so on may be cited as examples of the aforementioned curing agent. These curing agents may be used alone or in combinations of two or more types. Among these curing agents, an aliphatic diisocyanate is preferable.

Further, the adhesive film includes a base material film, and the base material film may be a film that has a 2% strain modulus of elasticity in each of a flow direction (an MD direction) and a width direction (a TD direction) of 190 to 600 MPa, and a tensile elongation in each of the flow direction (the MD direction) and the width direction (the TD direction) of 60% to 200%, for example.

A varnish that is known in the industry may be selected as appropriate for use on the front plate 30, but UV peeling varnish is preferable. The peel strength of the adhesive film F relative to the front plate 30 is 0.5 to 5 N / 220 mm, preferably 0.5 to 4 N / 220 mm, and more preferably 0.5 to 3 N / 220 mm. By setting the peel strength of the adhesive film F relative to the front plate 30 within a range of 0.5 to 5 N / 220 mm, the adhesive film F can adhere appropriately to the front plate 30 without being wound back even when the width of a gripping margin is narrow due to the reduced height of the front plate 30, and as a result, the adhesive film F can be gripped easily the next time the adhesive film F is used. Below 0.5 N / 220 mm, the adhesive force decreases such that the adhesive film F is wound back, making it difficult to extract the adhesive film F the next time the adhesive film F is used. Further, when the peel strength exceeds 5 N / 220 mm, the adhesive film F adheres too firmly to the front plate 30 such that the adhesive film F is difficult to grip, and as a result, the adhesive film F is more likely to peel away from the front plate 30 partially and tear vertically.

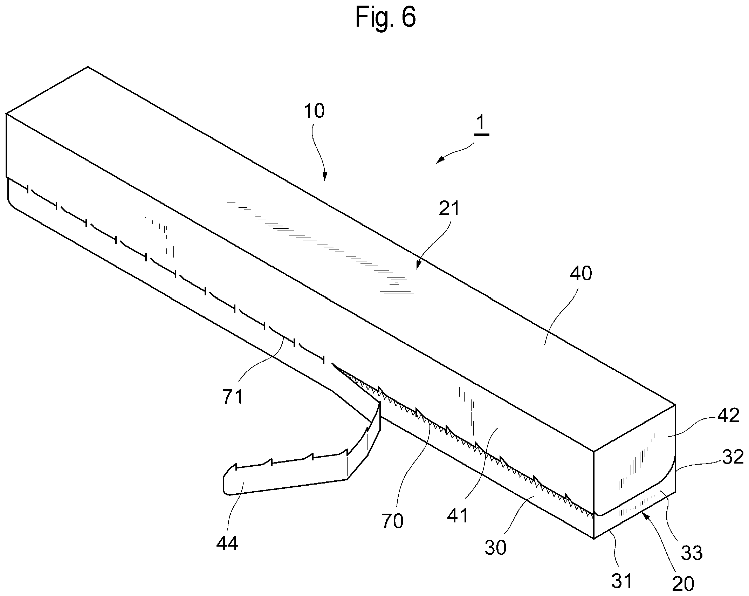

During use of the adhesive film storage device 1 configured as described above, first, as shown in Fig. 6, the adhesive film storage box 10 is opened by tearing away the opening piece 44. Next, the adhesive film F on the adhesive film roll 11 is pulled toward the front side of the adhesive film storage box 10. Then, with the cover 21 closed, as shown in Fig. 5, the adhesive film F is pulled upward relative to the adhesive film storage box 10, for example, such that the adhesive film F is cut to a predetermined length by the cutting blade 70.

According to this embodiment, the height H of the front plate 30 of the adhesive film storage box 10 is set to be lower than the back plate 32 and the side plates 33 and 50% to 90% of the outer diameter R of the core tube 60 of the adhesive film roll 11, and therefore, when the adhesive film F is pulled out of the adhesive film storage box 10, a situation in which the adhesive film F adheres to the front plate, thereby becoming difficult to pull, can be suppressed. As a result, with the adhesive film storage box 10 according to this embodiment, the adhesive film F can be pulled out easily during use while being stored appropriately. When the adhesive film F is an infection prevention film, the adhesive film F can be stored hygienically, which is particularly preferable.

Further, by setting the height H of the front plate 30 between 50% and 90% of the outer diameter R, an angle having a low peel strength is formed between the adhesive film F and the core tube 60, and therefore the adhesive film F can be pulled lightly. Note that when the height H of the front plate 30 exceeds 90% of the diameter of the core tube 60 of the adhesive film roll 11, it may be impossible to suppress adhesion of the adhesive film F to the front plate 30 sufficiently, and as a result, it may become impossible to pull out the adhesive film F. When the height H of the front plate 30 is less than 50% of the diameter of the core tube 60 of the adhesive film roll 11, meanwhile, the adhesive film roll 11 is more likely to jump out of the adhesive film storage box 10. Moreover, when cutting the adhesive film, the adhesive film F is more difficult to cut while sandwiched between the covering piece 41 and the front plate 30, and as a result, the adhesive film F is wound back after being cut, making it difficult to grip the end of the adhesive film F the next time the adhesive film F is used.

The folded-in portions 50 are provided on the side faces of the main body 20, and therefore the adhesive film roll 11 can be prevented from jumping out of the main body 20 even with the low front plate 30.

Incidentally, when the adhesive film F is pulled out of the adhesive film storage box 10, the adhesive film roll 11 may be pulled forward so as to impinge on the front plate 30, with the result that external force is exerted on the front plate 30. By making the height H of the front plate 30 lower than the side plates 33, the strength of the front plate 30 is reduced correspondingly. However, the adhesive film storage box 10 according to this embodiment includes the front plate side pieces 34 as reinforcing members for reinforcing the front plate 30, and therefore strength can be secured in the front plate 30 against the pressing force of the adhesive film roll 11. Hence, the front plate 30 can be prevented from bending and breaking even when the adhesive film F is pulled forcefully.

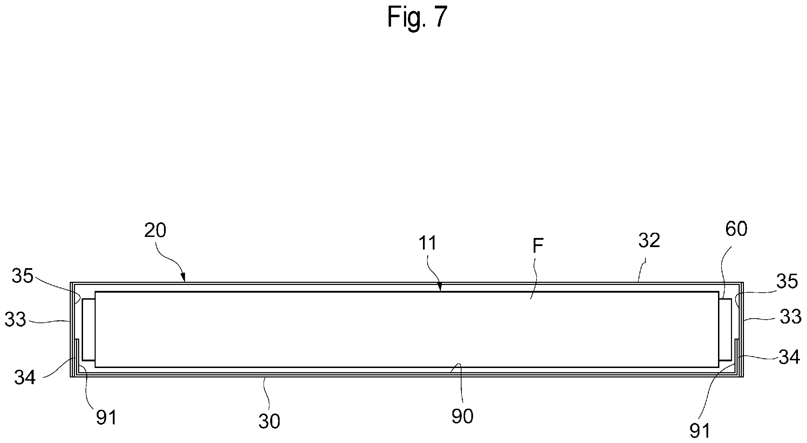

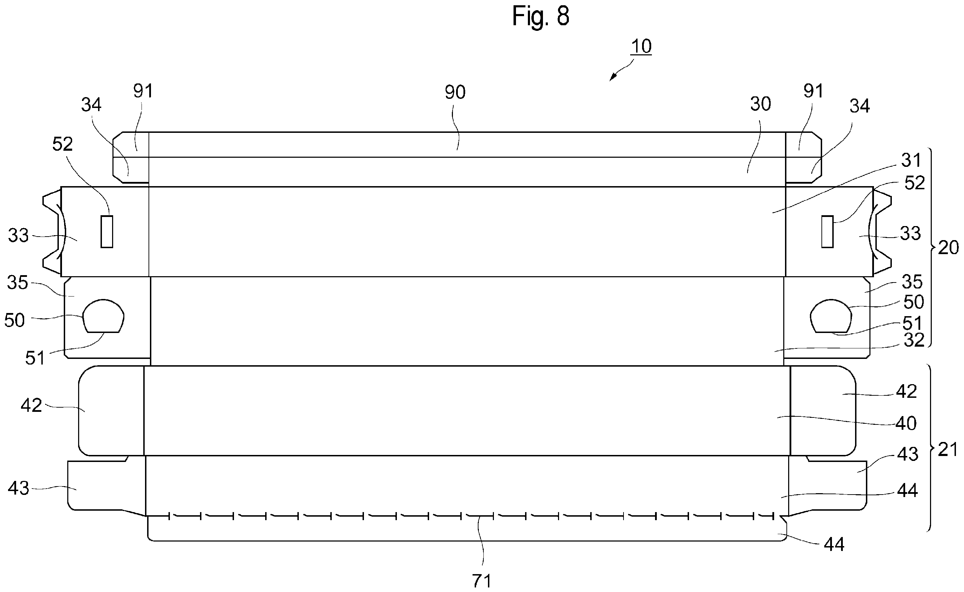

Note that in this embodiment, the front plate side pieces 34 are provided as the reinforcing members of the front plate 30, but the reinforcing members may have a different configuration. As shown in Figs. 7 and 8, for example, a reinforcing plate 90 may be provided on the rear surface of the front plate 30 as a reinforcing member. For example, the reinforcing plate 90 is formed in an elongated rectangular shape so as to extend from one longitudinal direction end of the front plate 30 to the other, and is adhered to the inner surface (the rear surface) of the front plate 30. The reinforcing plate 90 includes left and right side pieces 91 that are adhered respectively to the front plate side pieces 34, the side plates 33, or the back plate side pieces 35. Further, as shown in Fig. 8, the reinforcing plate 90 may be connected to the tip end of the front plate 30 and folded inward.

Note that the reinforcing plate 90 may have other shapes and structures, as long as the front plate 30 can be reinforced thereby.

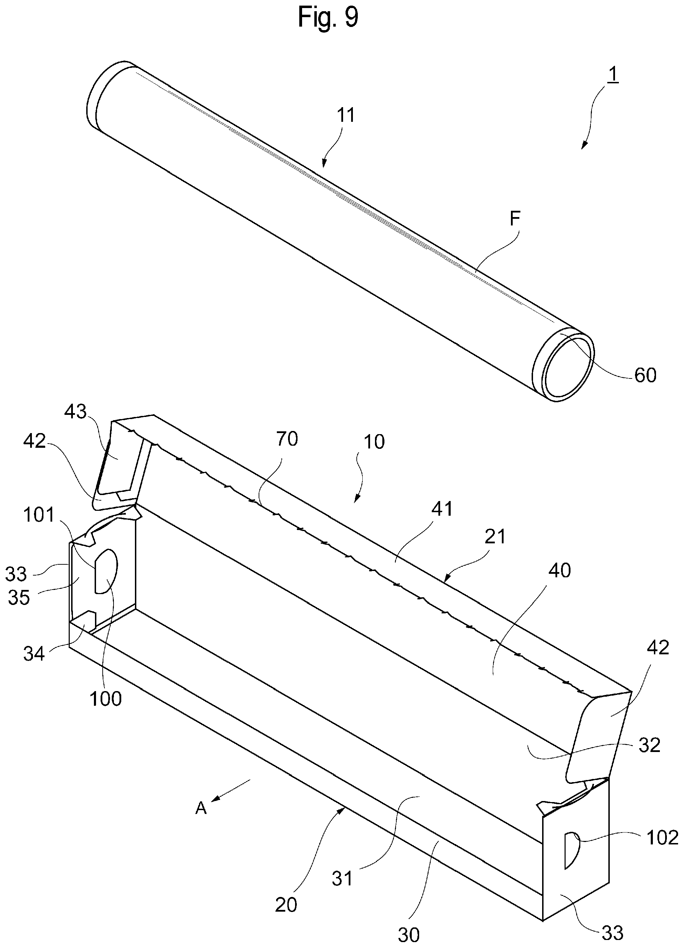

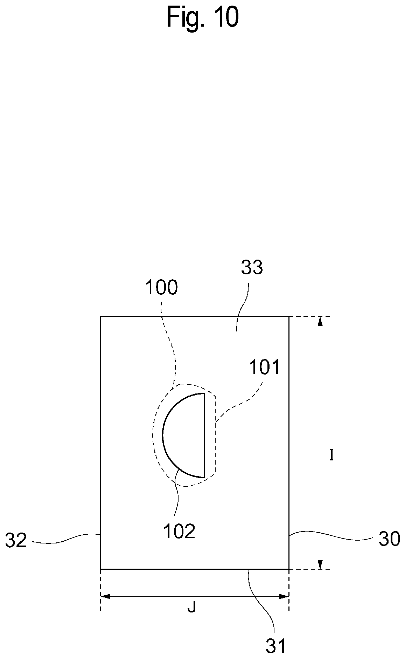

The jump-out suppressing member for preventing the adhesive film roll 11 from jumping out of the main body 20 may be configured to hold the adhesive film roll 11 so that the adhesive film roll 11 does not contact the bottom plate 31. In this case, as shown in Fig. 9, for example, a folded-in portion 100 serving as the jump-out suppressing member is formed in the center of each back plate side piece 35. Each folded-in portion 100 is formed in the shape of a tongue (a substantially semicircular shape) that projects toward the rear side, for example, and a fold line 101 is formed on a front side thereof. Further, as shown in Figs. 9 and 10, a substantially semicircular push-in piece 102 for pushing the folded-in portion 100 on the back plate side piece 35 from the outside in order to fold the folded-in portion 100 inward is formed on the side plate 33 overlapped onto the outside of the back plate side piece 35. The side plates 33 and the back plate side pieces 35 of the main body 20 are formed in a vertically elongated rectangular shape, for example, and the folded-in portions 100 and push-in pieces 102 are formed in positions sufficiently removed from the bottom plate 31.

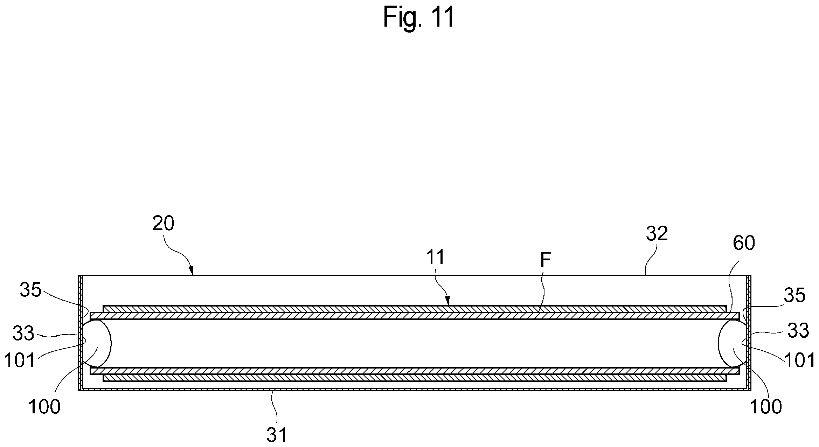

For example, the side plates 33 and the back plate side pieces 35 are formed such that a vertical length I thereof is at least 1.3 times a horizontal width J thereof. During manufacture of the main body 20, the folded-in portions 100 are not folded in, but after the adhesive film roll 11 is stored in the main body 20, the push-in pieces 102 are pushed from the outside, as shown in Fig. 11, so that the folded-in portions 100 are folded into the tube forming the core tube 60, whereby the adhesive film roll 11 is held by the folded-in portions 100 so as not to contact the bottom plate 31. As a result, the adhesive film roll 11 can be prevented from jumping out of the main body 20. In addition, the adhesive film roll 11 does not contact the bottom plate 31 and can therefore be rotated with low friction. Hence, the pull-out resistance of the adhesive film F (the force required to pull the adhesive film F) can be stabilized, leading to an improvement in the ease with which the adhesive film F is pulled. Note that the jump-out suppressing member is not limited to the configuration of the folded-in portion 100, and another configuration may be employed.

(Second Embodiment)

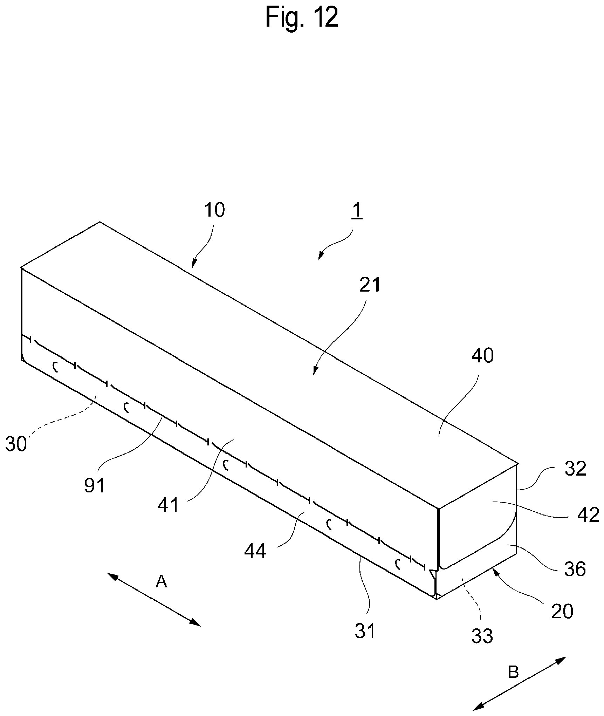

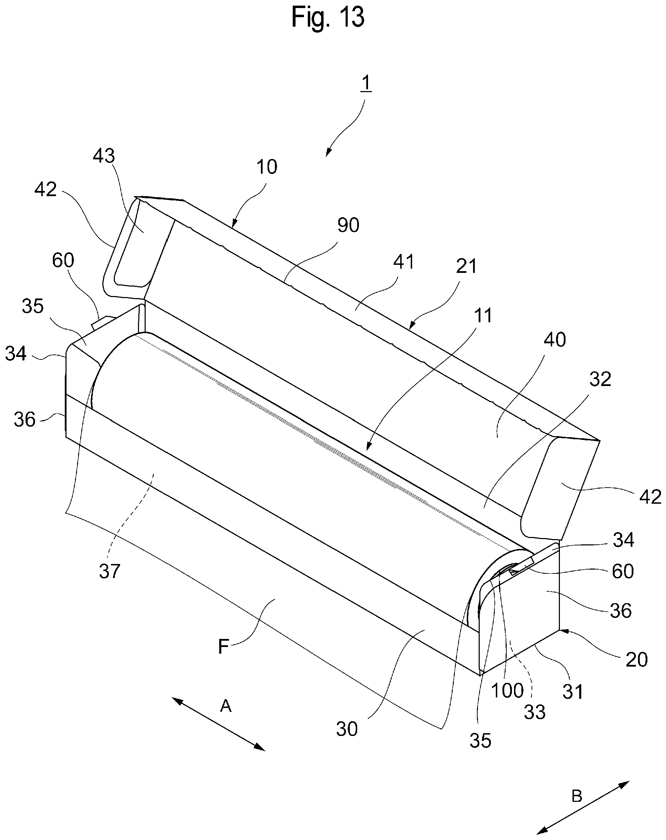

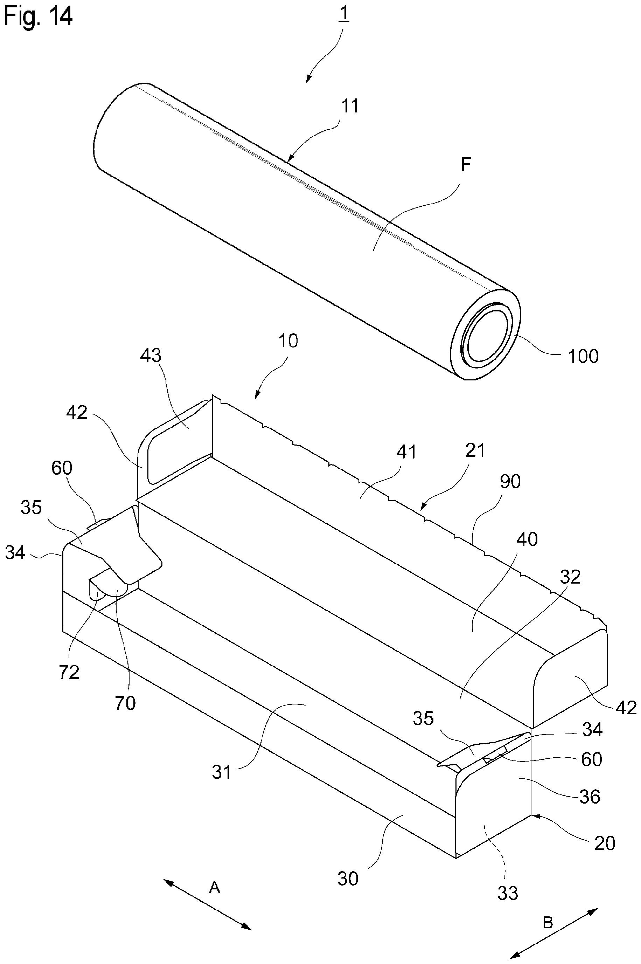

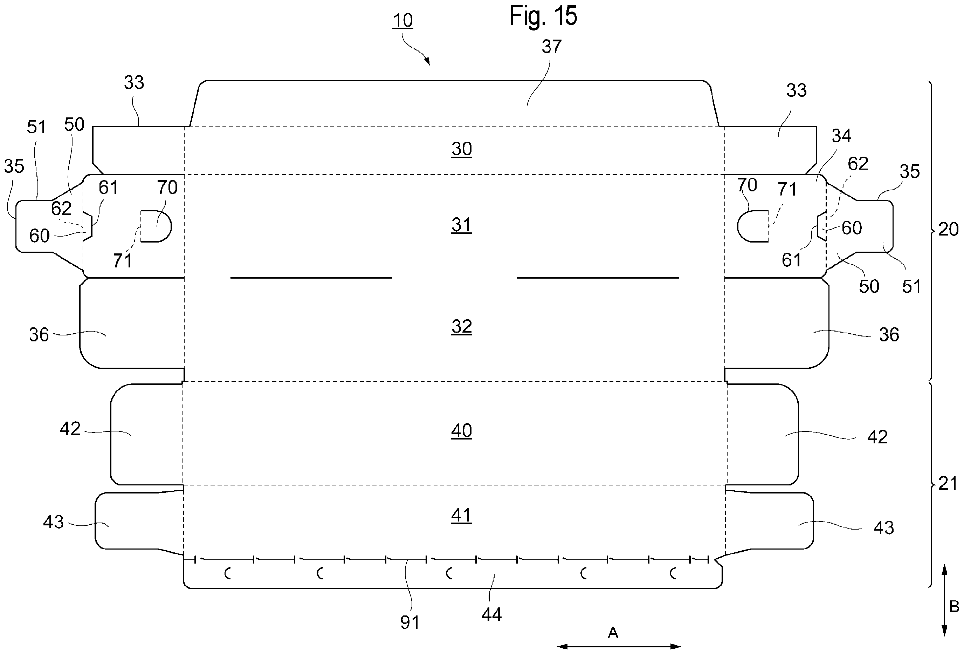

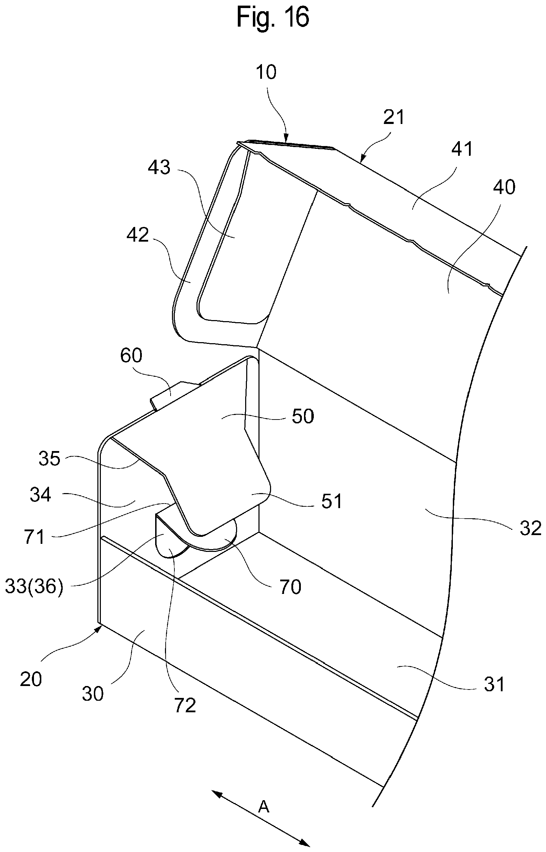

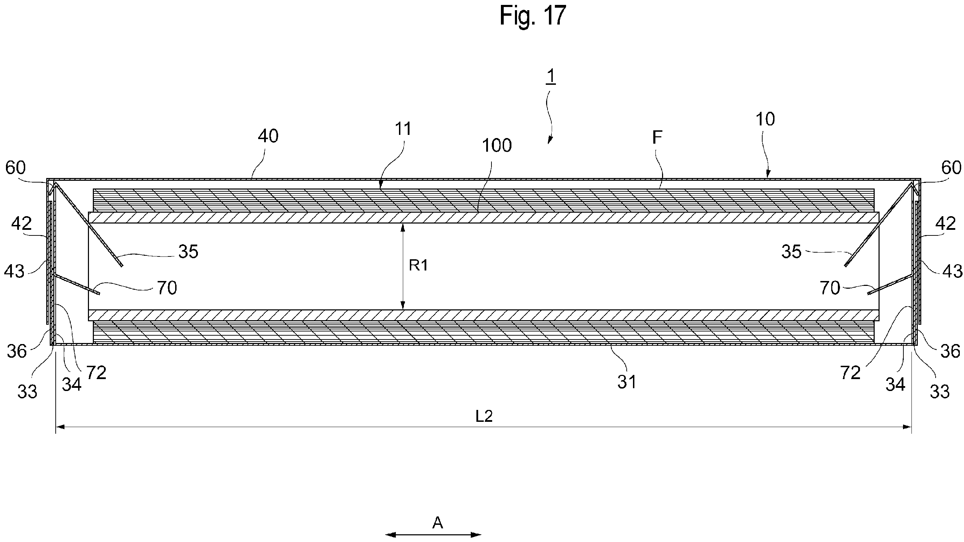

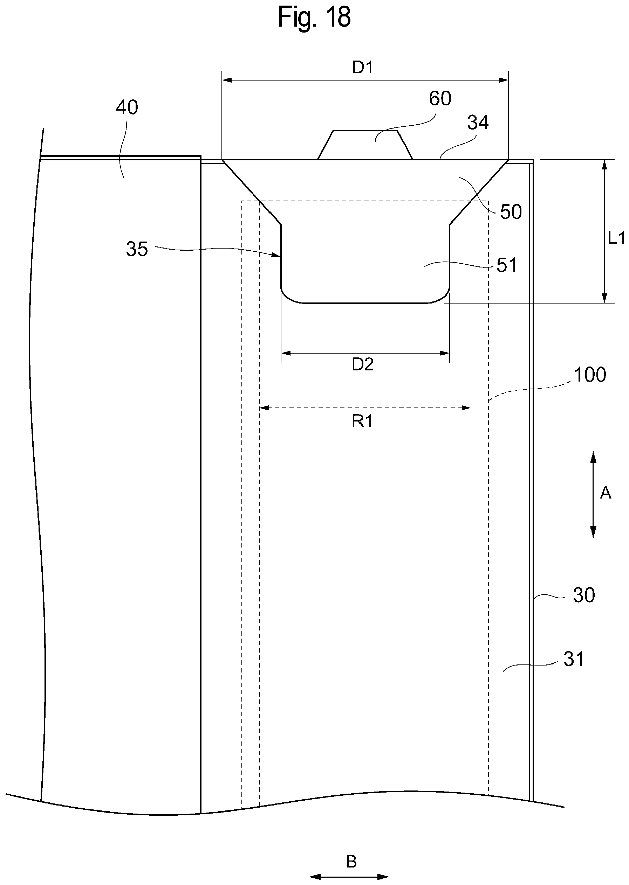

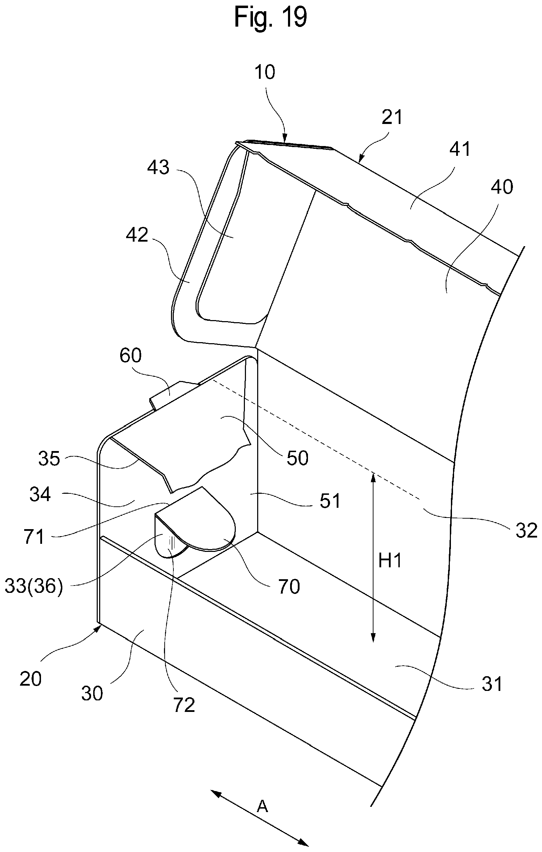

Fig. 12 is a perspective view showing an example of the adhesive film storage device 1 according to this embodiment. Fig. 13 is a perspective view showing the adhesive film storage device 1 in an open state.
The adhesive film storage device 1 includes the adhesive film storage box 10 and a roll 11 of the adhesive film F.
The adhesive film storage box 10 takes the overall shape of a rectangular parallelepiped, for example. As shown in Figs. 14 and 15, the adhesive film storage box 10 includes the main body 20 and the cover 21. The main body 20 includes, for example, the front plate 30, the bottom plate 31, the back plate 32, front plate side pieces 33, side plates 34 serving as first side plates, extension pieces 35, back plate side pieces 36, a folded-back rear plate 37, and so on. The cover 21 includes, for example, the cover plate 40, the covering piece 41, the cover plate side pieces 42, the covering side pieces 43, the opening piece 44, and so on.
As shown in Fig. 14, the main body 20 is formed in the shape of a rectangular parallelepiped with an open upper surface. The front plate 30, the bottom plate 31, the back plate 32, and the side plates 34 are each formed in a rectangular shape so as to constitute five surfaces of the main body 20.
As shown in Fig. 15, the back plate side pieces 36 are connected to respective ends of the back plate 32 in the longitudinal direction (an A direction in Fig. 15). The front plate side pieces 33 are connected to the respective longitudinal direction ends of the front plate 30. The folded-back rear plate 37 is connected to one end of the front plate 30 in the width direction (a B direction in Fig. 15). As shown in Fig. 14, each back plate side piece 36 is folded back to the side plate 34 side so as to cover the outer surface of the side plate 34, and adhered to the outer surface of the side plate 34. Each front plate side piece 33 is folded back to the side plate 34 side so as to be interposed between the back plate side piece 36 and the side plate 34, and adhered to the outer surface of the side plate 34. The folded-back rear plate 37 is folded back onto the rear surface of the front plate 30 and adhered to the rear surface of the front plate 30.
As shown in Fig. 15, the extension pieces 35 are formed in a substantially rectangular plate shape and connected respectively to longitudinal direction outside ends of the side plates 34 on the respective sides. As shown in Figs. 14, 16, and 18, the extension pieces 35 are folded into the interior of the main body 20 from the upper portions of the side plates 34 so as to extend toward a center side of the main body 20.
Each extension piece 35 includes a first part 50 connected to the upper end of the side plate 34 and a second part 51 connected to a tip end side of the first part 50. As shown in Fig. 18, the first part 50 is configured such that a base end (the part connected to the side plate 34) thereof has an equal width D1 to the side plate 34 and the width thereof gradually narrows toward the tip end side. The second part 51 has an identical width D2 to the tip end of the first part 50 over the entirety thereof (from the base end to the tip end). The width D1 is greater than an inner diameter R1 of a core tube 100, to be described below, of the roll 11, and the width D2 is smaller than the inner diameter R1. For example, a maximum width of the part of the extension piece 35 that is positioned inside the core tube 100 is set at 60% to 90% of the inner diameter R1 of the core tube 100. Further, a length L1 of the extension piece 35 is set at 10% to 15% of a longitudinal direction dimension L2 (shown in Fig. 17) of the interior of the main body 20, and 50% to 80% of a height direction dimension H1 (shown in Fig. 19) of the interior of the main body 20. As shown in Figs. 13 and 17, with the configuration described above, the extension piece 35 extends from the upper end of each side plate 34 into the interior of the core tube 100 of the roll 11 in the main body 20 so as to be able to push the roll 11 toward the center side from either end. Note that when the adhesive film storage device 1 is assembled, the roll 11 is stored in the main body 20 from above, as shown in Fig. 14, such that the extension pieces 35 enter the core tube 100 of the roll 11.
As shown in Fig. 16, a projecting portion 60 projecting to the outside of each of the side plates 34 is formed in the center of the upper end of each side plate 34. As shown in Fig. 15, the projecting portion 60 is formed by forming a slit 61 in the upper portion of the side plate 34 and raising a region on the inside of the slit 61. A dotted line-shaped cut line 62 is formed in a part serving as a base end of the projecting portion 60 (the upper end of the side plate 34), and the projecting portion 60 is bent outward. As shown in Fig. 17, with this configuration, the projecting portion 60 impinges on the inner surface of the cover 21 when the opening in the main body 20 is closed,
[0052]     A folded-in portion 70 is formed in the center of each side plate 34 so as to project into the interior of the main body 20. As shown in Fig. 19, the folded-in portion 70 is formed in the shape of a tongue (a substantially semicircular shape) that projects downward, for example, and a fold line 71 is formed on an upper end thereof. The folded-in portions 70 are folded into the core tube 100 of the roll 11 so that the roll 11 is held by the folded-in portions 70, and as a result, the roll 11 can be prevented from jumping out of the main body 20. When the folded-in portion 70 is folded in, an opening 72 is formed in the side plate 34, but this opening 72 is closed by the back plate side piece 36 or the front plate side piece 33 serving as a second side plate.

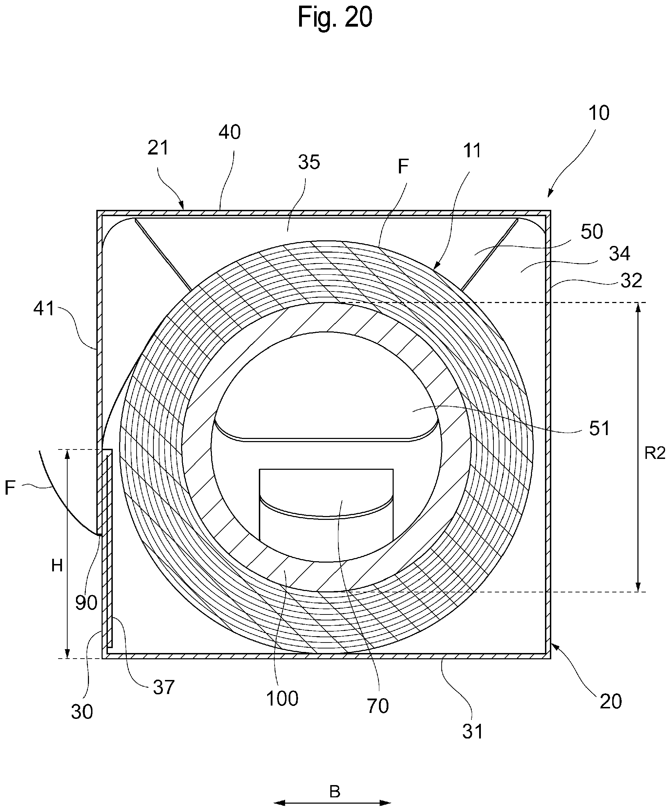

As shown in Figs. 14 to 16, the back plate 32 and the side plates 34 are formed at an approximately identical height, while the front plate 30 is formed to be lower than the back plate 32 and the side plates 34. The front plate 30 is formed in the shape of a rectangular plate having a constant height in the longitudinal direction of the adhesive film storage box 10. As shown in Fig. 20, the height H of the front plate 30 is set at 50% to 90%, preferably 55% to 85%, and more preferably 60% to 80% of an outer diameter R2 of the core tube 100 of the roll 11, for example. Further, the height H of the front plate 30 may be set at no more than half the height of the back plate 32 and the side plates 34. Furthermore, the vertical width of the front surface opening formed in the main body 20 by the reduced height of the front plate 30 may be set to equal or exceed half the height of the back plate 32 and the side plates 34.

As shown in Fig. 20, the cover plate 40 of the cover 21 extends frontward from the upper edge of the back plate 32 so as to cover the upper surface of the main body 20. The covering piece 41 extends toward the front surface (the front plate 30) side from the front edge of the cover plate 40. The tip end of the covering piece 41 overlaps the front plate 30 at least partially. As shown in Figs. 13 and 14, a saw blade-shaped cutting blade 90 is provided on the rear side of the tip end of the covering piece 41, for example, as the cutting portion for cutting the adhesive film F pulled off the roll 11 in the main body 20. A metal such as tin plate, or alternatively resin or paper, may be used as the material of the cutting blade 90.

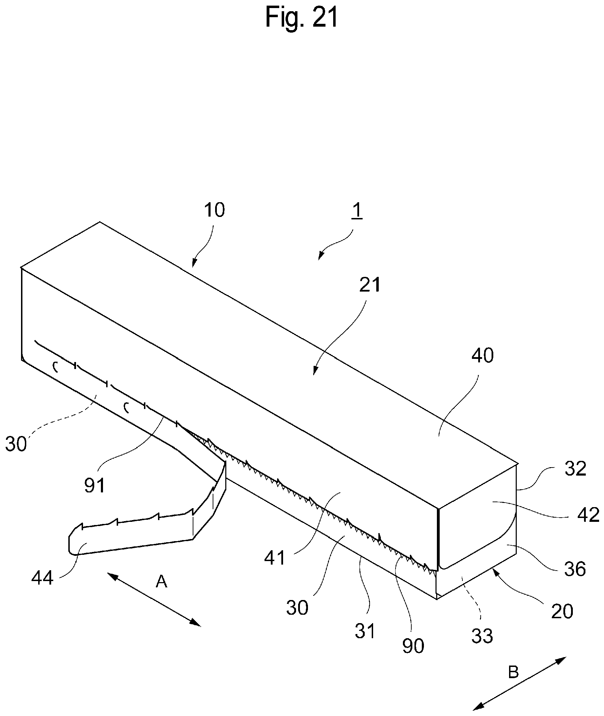

As shown in Fig. 12, before the adhesive film storage device 1 is used, the adhesive film storage box 10 is sealed by the opening piece 44 connected to the tip end of the covering piece 41 of the cover 21. The opening piece 44 is formed in the shape of a strip positioned on the front surface of the front plate 30 of the main body 20 so as to extend in the longitudinal direction of the adhesive film storage box 10. The opening piece 44 is connected tearably to the tip end of the covering piece 41 via a tear line 91 and adhered to a plurality of locations on the front surface of the front plate 30. As shown in Fig. 21, when the adhesive film storage box 10 is to be opened, the cover 21 can be opened by tearing the opening piece 44 in the longitudinal direction of the box.

Note that cardboard, coated cardboard, corrugated cardboard, and so on may be cited as examples of the base paper constituting the adhesive film storage box 10, but the present invention is not limited thereto, and any type of paper known in the industry may be selected as appropriate for use. The base paper may be formed by implementing embossing, printing, or laminating using polyethylene or the like on any of these materials.

As shown in Fig. 14, the roll 11 (an infection prevention film roll) is formed by winding the adhesive film F, which is a film having an adhesive property such as injection prevention film, for example, onto the core tube 100, which is cylindrical and made of paper. Note that infection prevention film is a film for preventing cross-infection among medical personnel in a hospital by preventing bacteria and viruses, as well as blood, vomit, and other bodily fluids, from adhering to keyboards of electronic charts, various testing equipment having operating consoles, and so on with which a plurality of medical personnel come into contact. The infection prevention film will be described in detail below.

The roll 11 may be formed such that the core tube 100 and the adhesive film F have identical lengths in the longitudinal direction A, whereby respective end faces of the core tube 100 and the adhesive film F are aligned, or such that the length of the core tube 100 in the longitudinal direction A is greater than the length of the adhesive film F in the longitudinal direction A, whereby the respective end faces of the core tube 100 and the adhesive film F are offset. In the former case, the roll 11 is manufactured at a predetermined length by winding the adhesive film F around the sufficiently long core tube 100 and then cutting the core tube 100, for example, and in the latter case, the roll 11 is manufactured by winding the adhesive film F around the core tube 100 that has been cut to a predetermined length in advance, for example. The roll 11 on which the adhesive film F is wound is configured such that when the surface area of the core tube 100 projected from above is set at 100%, the adhesive film F is wound so as to cover 90% to 100%, preferably 94% to 100%, and more preferably 97% to 100% of the surface area of the core tube 100. In so doing, the adhesive film storage box 10 can be provided in accordance with the width of the adhesive film F.

During use of the adhesive film storage device 1 configured as described above, first, as shown in Fig. 21, the adhesive film storage box 10 is opened by tearing away the opening piece 44. Next, the adhesive film F on the roll 11 is pulled toward the front side of the adhesive film storage box 10. Then, with the cover 21 closed, as shown in Fig. 20, the adhesive film F is pulled upward relative to the adhesive film storage box 10, for example, such that the adhesive film F is cut to a predetermined length by the cutting blade 90.

According to this embodiment, the adhesive film storage box 10 can be realized so as to exhibit superior qualities in terms of use and storage of the adhesive film F. Further, the main body 20 includes the extension pieces 35 extending into the main body 20 from the upper portions of the respective side plates 34, and the extension pieces 35 are configured to be capable of pushing the roll 11 in the main body 20 toward the center side. Hence, when the adhesive film F is pulled out of the adhesive film storage box 10, the extension pieces 35 retain the roll 11 on the center side, thereby suppressing movement of the roll 11 within the main body 20. Accordingly, the ends of the adhesive film F wound onto the roll 11 in the longitudinal direction A and the ends of the adhesive film F pulled from the roll 11 in the longitudinal direction A are prevented from contacting side wall faces of the adhesive film storage box 10. As a result, dust can be prevented from adhering to the adhesive film F. Moreover, the adhesive film F can be prevented from contacting and being damaged by the adhesive film storage box 10. Furthermore, soiling occurring when the adhesive adheres to the adhesive film storage box 10 can be prevented.

The extension pieces 35 are configured to be capable of extending into the core tube 100 of the roll 11 in the main body 20 and can therefore contact the roll 11 reliably so as to push the roll 11 toward the center side. Further, when the extension pieces 35 enter the core tube 100, a rotary axis of the roll 11 is stabilized, and therefore the adhesive film F can be pulled out smoothly. Moreover, the extension pieces 35 do not obstruct an operation to replace the roll 11, and therefore, when a problem such as the adhesive film F being wound back occurs, the roll 11 can be removed easily. Repair work can then be executed, whereupon the roll 11 can be mounted in the adhesive film storage box 10 again.

The extension pieces 35 are narrower in width at the tip end than at the base end, and can therefore be inserted into the core tube 100 of the roll 11 easily.

Each extension piece 35 includes the first part 50 connected to the upper end of the side plate 34 and the second part 51 connected to the first part 50. The first part 50 gradually narrows in width toward the tip end side, while the width of the second part 51 remains constant. Hence, the roll 11 can be pushed by the first part 50 and supported by the second part 51, and therefore the roll 11 can be pushed toward the center side appropriately.

The projecting portion 60 projecting to the outside from each side plate 34 is formed on the upper portion of each side plate 34, and the projecting portion 60 is configured to contact the inner surface of the cover 21 closing the opening in the main body 20. The projecting portion 60 therefore acts as a fixing member so that when the cover 21 is fully closed, the cover 21 is securely fixed to the main body 20. As a result, a situation in which the cover 21 opens during use such that dust enters the adhesive film storage box 10 and adheres to the adhesive film F can be suppressed.

The dotted line-shaped cut line 62 is provided on the base end of the projecting portion 60, and therefore the projecting portion 60 can be bent reliably outward so that the cover 21 is fixed appropriately when closed.

The side plate 34 includes the folded-in portion 70 that is folded into the core tube 100 of the roll 11 in the main body 20, and the opening formed in the side plate 34 when the folded-in portion 70 is folded is closed by the back plate side piece 36 or the front plate side piece 33. Hence, when the adhesive film F is pulled out, the roll 11 can be prevented from jumping out of the main body 20, and accordingly, dust can be prevented from entering the adhesive film storage box 10.

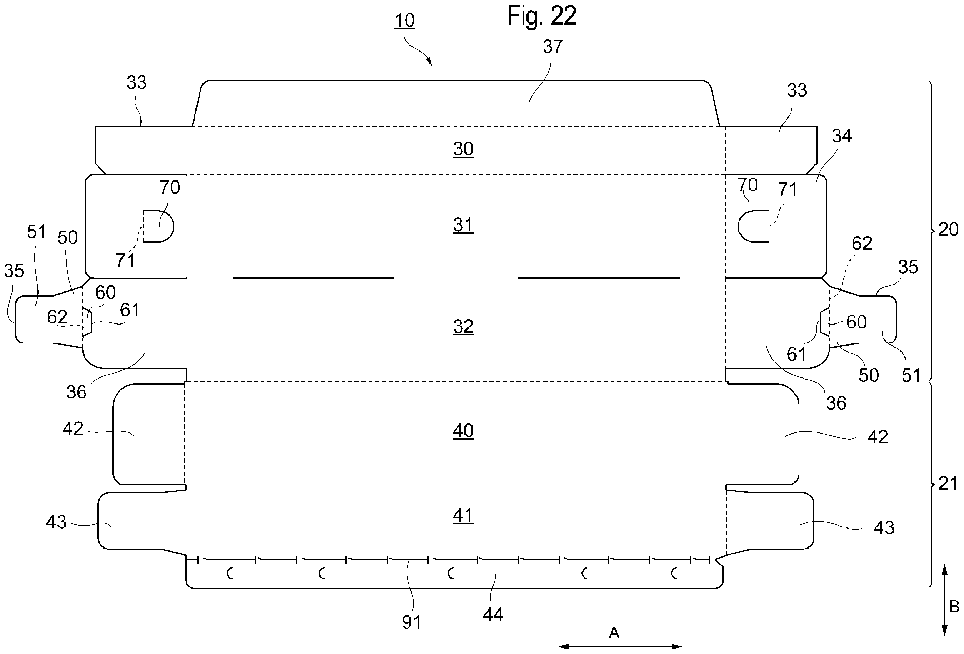

In the embodiment described above, the extension piece 35 extends from the upper end of the side plate 34, but may extend from another part forming the side wall. For example, the extension piece 35 may extend into the interior of the main body 20 from an upper end of the back plate side piece 36. In this case, as shown in Fig. 22, the extension piece 35 is connected to an outside end of the back plate side piece 36 on each side of the back plate 32,

Preferred embodiments of the present invention will be described below with reference to the figures. Identical elements have been allocated identical reference numerals, and duplicate description thereof has been omitted. Further, unless specified otherwise, positional relationships such as up, down, left, right, and so on are assumed to be based on the positional relationships shown in the figures. Moreover, the dimensional ratios in the figures are not limited to the illustrated ratios. Furthermore, the following embodiments are examples used to illustrate the present invention, and the present invention is not limited to these embodiments.

The overall configurations and shapes of the adhesive film storage box 10, for example, are not limited to those of the above embodiments. For example, the shape of the extension piece 35 is not limited to that of the above embodiment. The extension piece 35 may have a constant width from the base end to the tip end or gradually narrow in width from the base end to the tip end, for example. Further, the projecting portion 60 and the folded-in portion 70 may be omitted.

<Infection prevention film>

In the first embodiment and the second embodiment, an infection prevention film can be used as the adhesive film F. The infection prevention film includes a base material film that has a 2% strain modulus of elasticity in each of the flow direction (the MD direction) and the width direction (the TD direction) of 190 to 600 MPa, and a tensile elongation in each of the flow direction (the MD direction) and the width direction (the TD direction) of 60% to 200%. By including this base material film, the infection prevention film according to this embodiment is unlikely to stretch and break when used as a keyboard cover, for example, even after repeated key touches, and is therefore suitable for preventing infections.

The flow direction (MD direction) 2% strain modulus of elasticity of the base material film used in this embodiment is preferably between 190 and 550 MPa, more preferably between 200 and 500 MPa, and even more preferably between 200 and 400 MPa. Further, the width direction (TD direction) 2% strain modulus of elasticity of the base material film used in this embodiment is preferably between 190 and 550 MPa, more preferably between 200 and 500 MPa, and even more preferably between 200 and 400 MPa. Furthermore, the flow direction (MD direction) tensile elongation of the base material film used in this embodiment is preferably between 60% and 180%, more preferably between 65% and 170%, and even more preferably between 70% and 165%. Moreover, the width direction (TD direction) tensile elongation of the base material film used in this embodiment is preferably between 60% and 180%, more preferably between 65% and 170%, and even more preferably between 70% and 165%.

By employing a base material film having these characteristics, the infection prevention film according to this embodiment exhibits a tendency to be even more unlikely to stretch and break when used as a keyboard cover, for example, even after repeated key touches. Moreover, the film is similarly unlikely to stretch and break even on corner parts of various devices, and can therefore be used to protect the devices. Examples of the various devices include a multi-function printer in an office, a retail cash register or a backyard weighing machine, a switch or a console of hospital testing equipment such as MRI or CT equipment, an electronic chart trolley, a stretcher, and any other kind of device having corner parts.

Furthermore, by setting a density of the infection prevention film according to this embodiment in a range described below, the film becomes more resilient, and therefore the film does not become entangled when adhered, making the film even easier to handle.

There are no particular limitations on the method of controlling the 2% strain modulus of elasticity and the tensile elongation of the base material film to the ranges described above, but a method including the steps of extruding a resin and a plasticizer from an annular die as a single-layer or multilayer raw fabric, cooling and solidifying the extruded raw fabric, and executing stretching treatment on the cooled and solidified raw fabric may be cited as an example.

Furthermore, the base material film is preferably stretched. Stretching treatment is a well-known process for executing stretching treatment on a film raw fabric in one or two axial directions. There are no limitations on the stretching method, but a biaxial stretching method is preferable, and a consecutive or simultaneous biaxial stretching method or an inflation biaxial stretching method is more preferable. Among these methods, an inflation biaxial stretching method is preferable.

Note that in this embodiment, the 2% strain modulus of elasticity and the tensile elongation of the base material film can be measured using a method described in following examples.

The infection prevention film according to this embodiment preferably further includes an adhesive layer. Additionally, the adhesive force (the 180-degree peel strength compliant with JIS 0237) of the adhesive layer is preferably between 0.01 and 1.5 N / 25 mm, more preferably between 0.03 and 1.2 N / 25 mm, and even more preferably between 0.05 and 1.0 N / 25 mm. By further including this adhesive layer, the infection prevention film according to this embodiment adheres firmly to a keyboard when used as a keyboard cover, for example, but is also easy to peel off. Moreover, by making the resilient base material layer having the characteristics described above slightly adhesive using the adhesive layer, the film can be handled easily without becoming entangled when the film is adhered.

There are no particular limitations on the method of controlling the adhesive force of the adhesive layer to the range described above, but a method of adjusting the hardness of the adhesive or the thickness of the adhesive layer may be cited as an example.

Note that in this embodiment, the 180-degree peel strength, which is obtained using a measurement method compliant with JIS 0237, is used as the adhesive force of the adhesive layer.

There are no particular limitations on the adhesive used to form the adhesive layer, but an acrylic-based adhesive, a rubber-based adhesive, a silicone-based adhesive, and a urethane-based adhesive may be cited as examples, and among these adhesives, an acrylic-based adhesive or a urethane-based adhesive is preferable and an acrylic-based adhesive is particularly preferable.

With an acrylic-based adhesive, the cohesive force can be adjusted by adding a comonomer to the principal monomer, and the hardness of the adhesive can be adjusted by adding a monomer containing a functional group so as to provide a cross-linking point. In so doing, the target adhesive force can be realized. There are no particular limitations on the principal monomer, but various types of (meta-) alkyl acrylates, such as (meta-) methyl acrylate, (meta-) ethyl acrylate, (meta-) butyl acrylate, and (meta-) 2-ethylhexyl acrylate, may be cited as examples, and these monomers may be used individually or in combination. There are no particular limitations on the comonomer, but vinyl acetate, acrylonitrile, acrylamide, styrene, methyl metacrylate, and methyl acrylate may be cited as examples. There are no particular limitations on the functional group-containing monomer, but acrylate, hydroxyethyl acrylate, acrylamide, glycidyl methacrylate, and so on may be cited as examples. Furthermore, the adhesive force may be adjusted by adding rosin, a terpene, or a tackifier such as an aliphatic or aromatic synthetic resin. A stabilizer, an antioxidant, a photostabilizer, a UV absorber, and so on may also be added to the adhesive.

When an adhesive layer formed from the adhesive described above is provided, the effect by which the infection prevention film according to this embodiment adheres firmly to a keyboard when used as a keyboard cover, for example, but is also easy to peel off is realized to a greater extent. Moreover, the film can be handled more easily without becoming entangled.

Further, the thickness of the adhesive layer, from the viewpoint of the adhesive force of the adhesive layer, is preferably between 0.5 and 20 $\mu$m, more preferably between 1.0 and 17 $\mu$m, and even more preferably between 3 and 15 $\mu$m. When the thickness of the adhesive layer is set within this range, the effect by which the infection prevention film according to this embodiment adheres firmly to a keyboard when used as a keyboard cover, for example, but is also easy to peel off is realized to an even greater extent. Moreover, the film can be handled even more easily without becoming entangled.

Furthermore, a haze of a layer including the base material film and the adhesive layer of the infection prevention film according to this embodiment is preferably between 0.1 and 25, more preferably between 0.1 and 15, and even more preferably between 0.1 and 7. When the haze of the layer including the base material film and the adhesive layer is set within this range, the infection prevention film according to this embodiment is transparent enough for the article covered thereby to be visible.

There are no particular limitations on the method of controlling the haze of the layer including the base material film and the adhesive layer to this range, but a method of selecting the constituent components of the base material film and/or the adhesive layer and a plate used to apply the adhesive, and a method of appropriately selecting the smoothness of a release paper and the film, may be cited as examples.

Note that in this embodiment, the haze of the layer including the base material film and the adhesive layer can be measured using a method described in the following examples.

Further, the density of the infection prevention film according to this embodiment is preferably between 0.9 and 1.9 g/cm$^3$, more preferably between 1.2 and 1.9 g/cm$^3$, and even more preferably between 1.5 and 1.8 g/cm$^3$. By setting the density within this range, the infection prevention film according to this embodiment becomes more resilient, and as a result, the film can be handled even more easily without becoming entangled when the film is adhered.

There are no particular limitations on the method of controlling the density of the infection prevention film according to this embodiment within the range described above, but a method of appropriately selecting the constituent components of the base material film may be cited as an example. Moreover, two or more types of resin may be combined, and a filler may be used.

Note that in this embodiment, the density of the film can be measured using a method described in the following examples.

Furthermore, the thickness of the base material film is preferably between 5 and 50 $\mu$m, more preferably between 10 and 40 $\mu$m, and even more preferably between 15 and 30 $\mu$m. When the thickness of the base material film is set within this range, the infection prevention film according to this embodiment exhibits superior durability and is therefore even less likely to break. Hence, during use in a medical setting in particular, infections can be prevented even more effectively.

The heat shrinkage of the infection prevention film according to this embodiment at 120°C is preferably between 20% and 45% and more preferably between 25% and 40%. When the heat shrinkage at 120°C is set within this range, the infection prevention film according to this embodiment exhibits superior durability, and therefore, during use as a keyboard cover, for example, the infection prevention film is unlikely to stretch even after repeated key touches and is accordingly even less likely to break after catching on the corner parts of the keys. Hence, during use in a medical setting in particular, infections can be prevented even more effectively. Moreover, by setting the heat shrinkage at 120°C within this range, the infection prevention film according to this embodiment becomes more resilient, and therefore the film does not become entangled when adhered, making the film even easier to handle.

There are no particular limitations on the method of controlling the heat shrinkage of the infection prevention film according to this embodiment at 120°C to the range described above, but employing a base material film that has been stretched using a method including the aforesaid stretching treatment process may be cited as an example.

Note that in this embodiment, the heat shrinkage of the base material film at 120°C can be measured using a method described in the following examples.

The critical surface tension of the base material film at 20°C, as determined from the Zisman plot, is preferably between 35 and 50 mN/m and more preferably between 38 and 42 mN/m. By setting the critical surface tension of the base material film at 20°C, as determined from the Zisman plot, within this range, the adhesive can be applied more easily.

There are no particular limitations on the method of controlling the critical surface tension of the base material film at 20°C, as determined from the Zisman plot, to the range described above, but a method of controlling the critical surface tension mainly by adjusting the amounts of added resin material and plasticizer may be cited as an example.

Note that in this embodiment, the critical surface tension of the base material film at 20°C can be determined from the Zisman plot.

There are no particular limitations on the constituent components of the base material film, but a type of polyolefin such as polyvinylidene chloride, polyethylene, or polypropylene, a type of polyester such as polyethylene terephthalate, an ethylene vinyl alcohol copolymer, an ethylene vinyl acetate copolymer, polyamide, polyurethane, and so on, for example, may be applied as the material thereof. Among these materials, polyvinylidene chloride and electron beam-crosslinked polyethylene are preferable, and polyvinylidene chloride is more preferable. The polyvinylidene chloride preferably contains 100 parts by mass of a vinylidene chloride copolymer having a weight-average molecular weight of 70,000 to 140,000 and a vinylidene chloride monomer unit of 75% to 96%, and 1 to 10 parts by mass of a mixture of a fatty acid ester and an epoxy compound.

As the constituent components of the base material film, either a single component may be used alone, or two or more components may be used together.

The principal component of the base material film is particularly preferably a vinylidene chloride copolymer.

Note that in this embodiment, when the sum of the components is set at 100% by mass, the principal component occupies at least 70% by mass, preferably at least 75% by mass, more preferably at least 85% by mass, even more preferably at least 90% by mass, and particularly preferably 100% by mass.

The infection prevention film according to this embodiment may further include a release layer on the back surface of the base material film. When the infection prevention film according to this embodiment includes a release film, the infection prevention film tends to be easier to wind.

There are no particular limitations on the release layer, but a silicone-based layer or a long-chain alkyl pendant polymer-based layer, for example, can be used.

Further, the thickness of the release layer is preferably between 0.1 and 1.0 $\mu$m, more preferably between 0.15 and 0.9 $\mu$m, and even more preferably between 0.2 and 0.7 $\mu$m.

Furthermore, the infection prevention film according to this embodiment may be provided with a printing layer and a primer layer.

The infection prevention film according to this embodiment may further include a separator on the adhesive layer side of the layer including the base material film and the adhesive layer. There are no particular limitations on the separator, but a polyethylene terephthalate (PET) film subjected to silicone processing so as to become releasable, paper, or the like, for example, can be used.

Manufacturing method for infection prevention film>

There are no particular limitations on the method of manufacturing the infection prevention film according to this embodiment as long as the method includes a base material film that satisfies the 2% strain modulus of elasticity and tensile elongation ranges described above. Further, a base material film that satisfies the 2% strain modulus of elasticity and tensile elongation ranges described above can be manufactured using a method including the steps of extruding a resin and a plasticizer from an annular die as a single-layer or multilayer raw fabric, cooling and solidifying the extruded raw fabric, and executing stretching treatment on the cooled and solidified raw fabric, for example.

The stretching treatment step involves a well-known process for executing stretching treatment on a film raw fabric in one or two axial directions. There are no limitations on the stretching method, but using a biaxial stretching method is preferable, and using a consecutive or simultaneous biaxial stretching method or an inflation biaxial stretching method is more preferable. Among these methods, an inflation biaxial stretching method is preferable.

The infection prevention film according to this embodiment can be obtained by, for example, coating the base material film obtained as described above with an adhesive using a comma coater or a gravure coater, drying and then winding the resulting film, and then aging the film for two days at 40°. At this time, a method of coating the separator with the adhesive, drying the adhesive, and then laminating the base material film thereon may also be employed. Before applying the adhesive, the adhesive layer and the base material layer may be coated in advance with a compatible primer, and corona treatment may be implemented on the base material film. Further, back surface processing may be implemented on the base material in steps executed before and after the adhesive application step.

<Infection prevention film roll>

The infection prevention film roll according to this embodiment includes a core tube and the infection prevention film described above, which is wound onto the core tube. The adhesive film roll 11 or the roll 11 described in the first and second embodiments may be used as the infection prevention film roll.

The material of the core tube is not limited, but paper, plastic, metal, and so on, for example, may be used. The core tube may be cylindrical component having a hollow interior or a columnar component that is not hollow. Further, the diameter of the core tube is not limited, but may be set between 10 mm and 50 mm, for example.

In the infection prevention film roll according to this embodiment, the pull-out resistance required to pull the infection prevention film from the core tube is preferably between 30 and 300 cN / 200 mm width, more preferably between 35 and 250 cN / 200 mm width, and even more preferably between 40 and 200 cN / 200 mm width. By setting the pull-out resistance within this range, the required amount of infection prevention film can be pulled using light force.

There are no particular limitations on the method of controlling the pull-out resistance required to pull the infection prevention film from the core tube to the range described above, but setting the adhesive force of the infection prevention film or providing a release layer on the back surface of the infection prevention film may be cited as examples.

Note that in this embodiment, the pull-out resistance required to pull the infection prevention film from the core tube can be measured using a method described in the following examples.

<Infection prevention film storage box>

The infection prevention film storage box according to this embodiment is a box storing the infection prevention film roll described above, and includes pulling means for pulling the infection prevention film from the core tube. By including an extraction opening through which to pull the infection prevention film roll and ensuring that the roll can rotate freely inside the storage box, the required amount of film can be pulled out.

Further, the infection prevention film storage box according to this embodiment may include means for cutting the infection prevention film pulled from the core tube. In so doing, the required amount of pulled infection prevention film can be cut easily. A saw blade made of metal, paper, plastic, or the like, or a slide cutter may be used as the cutting means. The adhesive film storage box 10 described in the first and second embodiments can be used as the infection prevention film storage box.

Examples

Next, the present invention will be described more specifically, citing examples and comparative examples. Note, however, that as long as the spirit of the present invention is not exceeded, the present invention is not limited to the following examples.

The respective measurement methods used in the examples and comparative examples are as follows.

(1) 2% strain modulus of elasticity of base material film

The 2% strain modulus of elasticity was measured in accordance with ASTM-D882. More specifically, samples of the base material films of the respective examples were obtained by cutting each base material film to a width of 10 mm and a length of 100 mm in alignment with the MD and TD directions, whereupon measurement was implemented at a tensile speed of 5 mm/minute. A tangent was then drawn on an obtained stress-strain graph, whereupon a value obtained by converting a stress value at 2% elongation into a sample thickness was calculated. The average value of five measurements was set as a measurement value, and the average value of the MD direction and the TD direction was set as the value of the 2% strain modulus of elasticity. AUTOGRAPH AG-IS, manufactured by Shimadzu Corporation, was used as the measurement device.

(2) Tensile elongation of base material film

The tensile elongation was measured in accordance with ASTM-D882. More specifically, samples of the base material films of the respective examples were obtained by cutting each base material film to a width of 10 mm and a length of 100 mm in alignment with the MD and TD directions, whereupon measurement was implemented at a tensile speed of 300 mm/minute. The elongation at the point of breakage was obtained by setting the average value of five measurements as a measurement value and setting the average value of the MD direction and the TD direction as the value of the tensile elongation. AUTOGRAPH AG-IS, manufactured by Shimadzu Corporation, was used in the measurement.

(3) Adhesive force of adhesive layer

The adhesive force of the adhesive layer was measured as follows in compliance with JIS Z0237. More specifically, a sample was obtained by cutting the film to a width of 25 mm and a length of 300 mm in alignment with the MD direction, whereupon the film was adhered by causing a 2 kg weighted roller made of SUS304 plate and mirror-finished to make two return journeys over the film at 10 mm/second. A tension measurement was then implemented at a speed of 300 mm/minute, and the peel strength was obtained by setting the average value of five measurements as the measurement value. AUTOGRAPH AG-IS, manufactured by Shimadzu Corporation, was used in the measurement. The 180-degree peel strength obtained in this manner was set as the adhesive force of the adhesive layer.

(4) Haze of layer including base material film and adhesive layer Measurement was implemented in compliance with JIS K7136, and the average value of five measurements was set as the haze value.

$$\text{Haze (\%)} = \text{diffuse transmittance / total light transmittance} \times 100$$

A haze meter manufactured by Nippon Denshoku Industries Co., Ltd. was used in the measurement.

(5) Density of film

The density of the film was measured using the A method (underwater substitution method) on the basis of a method for measuring the density and specific gravity of plastic and non-foamed plastic, as defined by JIS K7112.

(6) Thickness of base material film

The film was cut using a microtome, whereupon the thickness of the base material film was measured by observing the cut cross-section using an optical microscope (VHX-900, manufactured by Keyence Corporation). The same measurement was performed at 1 m intervals in the MD direction (the winding direction) of the film, and finally, the average value of ten points was set as the thickness of the base material film.

(7) Heat shrinkage of film at 120°C

A sample was obtained by cutting the film to 10 cm square, whereupon the sample was left in an oven set at 120°C for one minute and then removed. The rate of change in the MD direction and the TD direction was measured, and the average value of ten measurements was set as the value of the heat shrinkage.

(8) Critical surface tension of base material film at 20°C

The critical surface tension of the base material film at 20°C was determined as follows from the Zisman plot. The contact angle was measured in relation to a liquid having a known surface tension and used in the Zisman plot, whereupon the surface tension was plotted on the x axis and $\cos\theta$ of the contact angle was plotted on the y axis. A value obtained by extrapolating a straight line obtained on the basis of this plotting to $\cos\theta = 1$ was set as the critical surface tension. Heptane, which is a type of n-alkane, decane, ethanol, which is a type of alcohol, ethylene glycol, 1-chlorohexane, which is a type of alkyl halide, or 1-choloroctane was used as the liquid having a known surface tension. A contact angle gauge DM-501, manufactured by Kyowa Interface Science Co., Ltd., was used in the measurement.

(9) Pull-out resistance required to pull film from core tube

A sample obtained by winding 20 m of a film having a width of 200 mm onto a paper tube was aged for one week at 28°C. The sample was then fitted into a rotatable fixture formed by coupling an acrylic cylinder to a steel rod having a diameter of 12 mm via a bearing, whereupon the steel rod was fixed to a tension testing machine so that the acrylic cylinder and the film wound onto the paper tube were capable of rotating freely. The end of the film was fixed to a plate having a width of 250 mm, which was joined directly to a load cell of the tension testing machine, and a load generated when the film was pulled at a speed of 1000 mm/minute was set as the pull-out resistance. The average value of five measurements was set as a measurement value. Product name "AUTOGRAPH AG-IS" (manufactured by Shimadzu Corporation) was used in the measurement.

(10) Durability of infection prevention film

The durability in a case where the infection prevention films obtained respectively in the examples and comparative examples were used as keyboard covers and keys were touched repeatedly in order to type 20,000 characters of random Japanese text was evaluated as follows according to the extent to which the films caught on the corner parts of the keys and broke.

<Evaluation reference of durability>

○: Fewer than five stretched parts
Δ: At least five stretched parts
X: At least one part that was partially broken due to stretching

(11) Resilience of infection prevention film

In a case where the infection prevention films obtained respectively in the examples and comparative examples were used as keyboard covers, an operation for unwinding the film from the film roll and adhering the film to a keyboard was executed 100 times, whereupon entanglement of the film was evaluated as follows.
○: Film becomes entangled and cannot be adhered to keyboard 5 times or less
Δ: Film becomes entangled and cannot be adhered to keyboard 6 to 19 times
X: Film becomes entangled and cannot be adhered to keyboard 20 times or more

(12) Peel strength of adhesive film from front plate

A front plate having a width of 220 mm was coated with UV peeling OP varnish (manufactured by Toyo Ink Co., Ltd.), whereupon the infection prevention films obtained respectively in the examples and comparative examples, each of which has a width of 220 mm, were adhered to the front plate. A central portion of each film was then gripped and pulled so as to be peeled away at a speed of 400 mm/minute. A load generated at that time was measured as the peel strength, and the average value of three measurements was set as a measurement value. Product name "AUTOGRAPH AG-IS" (manufactured by Shimadzu Corporation) was used in the measurement.

[Example 1]

[Manufacture of base material film]

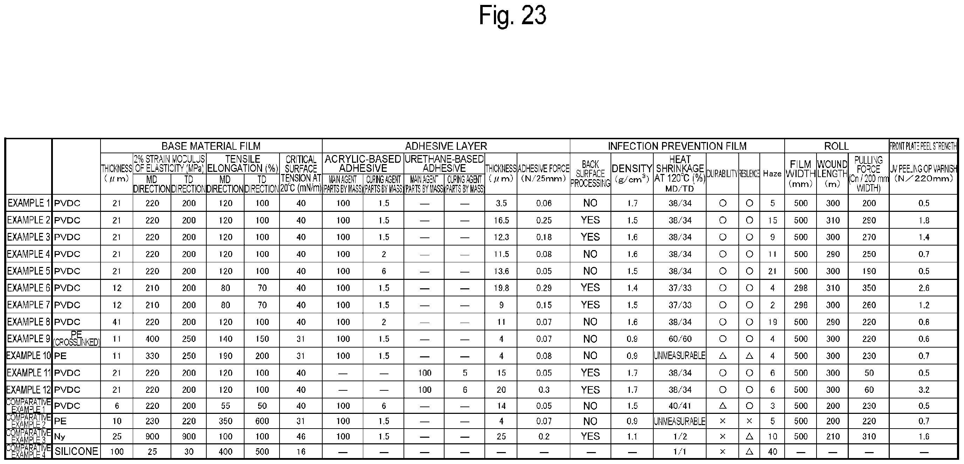

A base material film (a PVDC film, thickness: 21 $\mu$m) having a vinylidene chloride copolymer (PVDC) as the principal component was manufactured as follows. 3 parts by mass of dibutyl sebacate, serving as a fatty acid ester, 3 parts by mass of acetyl tributyl citrate, and 2 parts by mass of epoxidized soybean oil, serving as an epoxy compound, were mixed into 100 parts by mass of a vinylidene chloride-vinyl chloride copolymer (vinylidene chloride monomer unit content / vinyl chloride monomer unit content = 80% by mass / 20% by mass, weight-average molecular weight 120,000; denoted in Fig. 23 as "PVDC") for 5 minutes using a Henschel mixer. A pipe-shaped film obtained by extruding the obtained mixture in a pipe shape using a melt extruder, super-cooling the extruded mixture in a bath of cold water at approximately 10°C, passing the cooled mixture through 35°C water, and then subjecting the result to inflation biaxial stretching to a multiple of 3.0 in the longitudinal (MD) direction and a multiple of 4.0 in the width (TD) direction at a stretching temperature of 30°C was folded by a pinch roll, whereby a flat, elongated film with a thickness of 42 $\mu$m was manufactured. A 21 $\mu$m film was then obtained by peeling the film to a single film using a slitter. The characteristics of the obtained film (the base material film) were then measured using the methods described above. The measurement results are shown in Fig. 23.

[Manufacture of infection prevention film]

An infection prevention film was manufactured by coating a separator (a silicone-processed polyethylene terephthalate (PET) film) with an acrylic-based adhesive (main agent: X-314 (100 parts by mass), curing agent: K-315 (1.5 parts by mass), manufactured by Saiden Chemical Industry Co., Ltd.) to a thickness of 3.5 $\mu$m using a comma coater, laminating the base material film described above thereto, winding the resulting film and then aging the film for two days at 40°C, and then delaminating the separator. The characteristics of the obtained infection prevention film were then measured using the methods described above. The measurement results are shown in Fig. 23.

[Manufacture of infection prevention film roll]

An infection prevention film roll was manufactured by slitting the infection prevention film (width: 500 mm, length: 300 mm) manufactured as described above to a width of 200 mm and winding the film onto a cylindrical core tube. In the obtained infection prevention film roll, the pull-out resistance required to pull the film from the core tube was 200 cN / 200 mm width.

[Examples 2 to 8 and comparative example 1]

The thickness of the base material film, the mass ratio between the main agent and the curing agent of the adhesive, the thickness of the adhesive layer, and so on were modified as shown in Fig. 23. Otherwise, the infection prevention film and the infection prevention film roll were manufactured in a similar manner to example 1.

[Examples 11 and 12]

A polyurethane adhesive (main agent: SH-101 (100 parts by mass), curing agent: T-501B (5 to 6 parts by mass), manufactured by Toyo Ink Co., Ltd.) was used as the adhesive, as shown in Fig. 23. Otherwise, the infection prevention film and the infection prevention film roll were manufactured in a similar manner to example 1.

Back surface processing was implemented on the infection prevention film by applying a long-chain alkyl pendant polymer (Piroiru, manufactured by Lion Specialty Chemicals Co., Ltd.) at a thickness of 0.2 $\mu$m using a gravure coater.

[Example 9]

A tubular raw fabric having a folded width of 120 mm and a thickness of 500 $\mu$m was manufactured by extruding a substance obtained by adding 0.5% by mass of glycerin monooleate to a polyethylene-based resin (composition) in which LL (ethylene-1-octane copolymer, density 0.926 g/cm$^3$, MI 2.0 g/10 minutes): LD (high-pressure-processed, low-density polyethylene, density 0.921 g/cm$^3$, MI 0.4 g/10 minutes) = 70 : 30 from an annular die and then cooling and solidifying the result in cold water. Crosslinking treatment was then executed by introducing the resulting component into an electron beam irradiation device and irradiating the component with electron beams accelerated to 500 kV so as to obtain an absorbed dose of 80 kGy. A two-ply film was then obtained by introducing the resulting component into a stretching machine, reheating the component, passing the component through two pairs of differential nip rollers, forming bubbles therein by injecting air, and implementing inflation biaxial stretching under stretching conditions of a multiple of 8 in the MD direction and a multiple of 6 in the TD direction. The base material film was then obtained by peeling the two-ply film to a single film in a slitting process.

Apart from using the obtained base material film, the infection prevention film and the infection prevention film roll were manufactured in a similar manner to example 1. The characteristics of the obtained infection prevention film and infection prevention film roll were then measured using the methods described above. The measurement results are shown in Fig. 23.

[Example 10]

A raw fabric having a thickness of 130 $\mu$m was manufactured by extruding a five-layer structure constituted by EVA (ethylene vinyl acetate copolymer) / EVA, LL (ethylene-1-octane copolymer), VL (very low density polyethylene) / PP (polypropylene) / EVA, LL, and VL / EVA from a circular multilayer die increased in temperature to 220°C, rapidly cooling the result in cold water, and squeezing and folding the resulting laminated body in a pinch roll after applying a sodium oleate solution to the inner side thereof. The folded raw fabric was formed in a tubular shape by injecting air therein, then heated to 50°C, then subjected to inflation biaxial stretching to a multiple of 3.7 in a vertical direction and a multiple of 4.3 in a horizontal direction, and then picked up by a nip roll. Next, heat treatment was implemented by blowing 65°C hot air so as to realize the target thickness of the final film, which was 10 $\mu$m, whereupon the two ends of the film were cut and the film was wound as two single films.

Apart from using the obtained single film as the base material film, the infection prevention film and the infection prevention film roll were manufactured in a similar manner to example 1. The characteristics of the obtained infection prevention film and infection prevention film roll were then measured using the methods described above. The measurement results are shown in Fig. 23.

[Comparative example 2]

A base material film was manufactured by extruding LD (high-pressure-processed, low-density polyethylene, density 0.921 g/cm$^3$, MI 0.4 g/10 minutes) in a tubular shape using a melt extruder, forming bubbles therein, and then winding the result.

Apart from using the obtained base material film, the infection prevention film and the infection prevention film roll were manufactured in a similar manner to example 1. The characteristics of the obtained infection prevention film and infection prevention film roll were then measured using the methods described above. The measurement results are shown in Fig. 23.

[Comparative example 3]

A base material film having a thickness of 25 $\mu$m was obtained by extruding 6-nylon using a melt extruder and subjecting the result to biaxial stretching.

Apart from using the obtained base material film, the infection prevention film and the infection prevention film roll were manufactured in a similar manner to example 1. The characteristics of the obtained infection prevention film and infection prevention film roll were then measured using the methods described above. The measurement results are shown in Fig. 23.

[Comparative example 4]

A sheet having a thickness of 0.1 mm was created using two-liquid-based silicone rubber by kneading the rubber and leaving it overnight, and thus a base material film was obtained.

Apart from using the obtained base material film, the infection prevention film and the infection prevention film roll were manufactured in a similar manner to example 1. The characteristics of the obtained infection prevention film and infection prevention film roll were then measured using the methods described above. The measurement results are shown in Fig. 23. Industrial Applicability

The present invention is useful for providing an adhesive film storage box that can store an adhesive film so that the adhesive film can be pulled out easily during use. The present invention is useful for providing an adhesive film storage box and an adhesive film storage device that exhibit superior qualities in terms of use and adhesive film storage, and with which an adhesive film can be pulled out for use so that no dust adheres thereto.

Reference Signs List

| 1 | Adhesive film storage device |
| 10 | Adhesive film storage box |
| 11 | Adhesive film roll |
| 20 | Main body |
| 21 | Cover |
| 30 | Front plate |
| 32 | Back plate |
| 33 | Side plate |
| 40 | Cover plate |
| 41 | Covering piece |
| 60 | Core tube |
| 70 | Cutting blade |
| F | Adhesive film |

**Claims**

1. An adhesive film storage box for storing a roll of wound adhesive film having an adhesive property, the adhesive film storage box comprising:

   a main body having at least a front plate, a bottom plate, a back plate, and side plates, and having an open upper surface; and
   a cover capable of opening and closing an opening in the upper surface of the main body, wherein the cover has at least:

   a cover plate connected to an upper edge of the back plate of the main body so as to oppose the upper surface of the main body; and
   a covering piece connected to a front edge of the cover plate so as to oppose a front surface of the main body, a cutting portion capable of cutting the adhesive film is provided on a lower edge of the covering piece, and a height of the front plate is set to be lower than a height of the back plate and the side plates and to be between 50% and 90% of an outer diameter of a core tube of the roll.

2. The adhesive film storage box according to claim 1, wherein
   the adhesive film has an adhesive force (a 180-degree peel strength compliant with JIS 0237) between 0.01 and 1.5 N / 25 mm.

3. The adhesive film storage box according to claim 1 or 2, wherein
   a peel strength of the adhesive film relative to the front plate is between 0.5 and 5 N / 220 mm.

4. The adhesive film storage box according to any of claims 1 to 3, wherein
   a pull-out resistance for pulling the adhesive film from the core tube is between 30 and 300 cN / 200 mm width.

**5.** The adhesive film storage box according to any of claims 1 to 4, wherein
the adhesive film storage box stores the roll which is formed by winding the adhesive film such that when a surface area of the core tube projected from above is set at 100%, 97% to 100% of the surface area of the core tube is covered by the adhesive film.

**6.** The adhesive film storage box according to any of claims 1 to 5, wherein
the main body has an extension piece that extends into the main body from an upper portion of each side wall on each longitudinal direction side of the main body, and
the extension piece is configured to be capable of pushing the roll inside the main body toward a center side of the main body.

**7.** The adhesive film storage box according to claim 6, wherein
the extension piece is capable of extending into the core tube of the roll inside the main body.

**8.** The adhesive film storage box according to claim 7, wherein
the extension piece is narrower in width at a tip end than at a base end.

**9.** The adhesive film storage box according to claim 7 or 8, wherein
the extension piece has a first part connected to an upper end of the side wall and a second part connected to the first part,
the first part gradually decreases in width toward a tip end side, and the second part has an unvarying width.

**10.** The adhesive film storage box according to any of claims 6 to 9, wherein
the side wall has at least a first side plate facing an interior of the main body and a second side plate positioned on an outside of the first side plate,
the first side plate has a folded-in portion that enters the core tube of the roll in the main body,
the folded-in portion is formed by folding a part surrounded by a cut line formed in the first side plate inward, and
an opening in the first side plate, which is formed by folding the folded-in portion, is closed by the second side plate.

**11.** An adhesive film storage device in which the roll is stored in the adhesive film storage box according to any of claims 1 to 10.

**12.** An infection prevention film comprising
a base material film that has a 2% strain modulus of elasticity in each of a flow direction (an MD direction) and a width direction (a TD direction) of 190 to 600 MPa, and a tensile elongation in each of the flow direction (the MD direction) and the width direction (the TD direction) of 60% to 200%.

**13.** The infection prevention film according to claim 12, further comprising
an adhesive layer, wherein an adhesive force of the adhesive layer (a 180-degree peel strength compliant with JIS 0237) is between 0.01 and 1.5 N / 25 mm.

**14.** The infection prevention film according to claim 13, wherein
a haze of a layer having the base material film and the adhesive layer is between 0.1 and 25.

**15.** The infection prevention film according to any one of claims 12 to 14, wherein
a density is between 0.9 and 1.9 g/cm$^3$ and a thickness of the base material film is between 5 and 50 $\mu$m.

**16.** The infection prevention film according to any one of claims 12 to 15, wherein
a heat shrinkage at 120°C is between 20% and 45%.

**17.** The infection prevention film according to any one of claims 12 to 16, wherein
a principal component of the base material film is a vinylidene chloride copolymer.

**18.** The infection prevention film according to claim 13 or 14, wherein
a separator is provided on the adhesive layer side of a layer having the base material film and the adhesive layer.

**19.** An infection prevention film roll comprising:

a core tube; and
the infection prevention film according to any one of claims 12 to 18, which is wound onto the core tube.

20. The infection prevention film roll according to claim 19, wherein
a pull-out resistance for pulling the infection prevention film from the core tube is between 30 and 300 cN / 200 mm width.

21. An infection prevention film storage box for storing the infection prevention film roll according to claim 19 or 20, the infection prevention film storage box comprising
means for pulling the infection prevention film from the core tube.

22. An infection prevention film storage box for storing the infection prevention film roll according to claim 19 or 20, the infection prevention film storage box comprising
means for cutting the infection prevention film pulled from the core tube.

Fig. 1

# Fig. 2

# Fig. 3

Fig. 4

EP 3 486 195 A1

# Fig. 5

## Fig. 6

EP 3 486 195 A1

Fig. 7

Fig. 8

# Fig. 9

# Fig. 10

Fig. 11

# Fig. 12

# Fig. 13

Fig. 14

Fig. 15

EP 3 486 195 A1

# Fig. 16

# Fig. 17

EP 3 486 195 A1

# Fig. 18

# Fig. 19

# Fig. 20

# Fig. 21

Fig. 22

EP 3 486 195 A1

# Fig. 23

| | | BASE MATERIAL FILM | | | | | | ADHESIVE LAYER | | | | | | INFECTION PREVENTION FILM | | | | | | ROLL | | | FRONT PLATE PEEL STRENGTH |
| | | THICKNESS (μm) | 2% STRAIN MODULUS OF ELASTICITY (MPa) | | TENSILE ELONGATION (%) | | CRITICAL SURFACE TENSION AT 20°C (mN/m) | ACRYLIC-BASED ADHESIVE | | URETHANE-BASED ADHESIVE | | THICKNESS (μm) | ADHESIVE FORCE (N/25mm) | BACK SURFACE PROCESSING | DENSITY (g/cm³) | HEAT SHRINKAGE AT 120°C (%) MD/TD | DURABILITY | RESILIENCE | Haze | FILM WIDTH (mm) | WOUND LENGTH (m) | PULLING FORCE (Cn / 200 mm WIDTH) | UV PEELING OP VARNISH (N/220mm) |
| | | | MD DIRECTION | TD DIRECTION | MD DIRECTION | TD DIRECTION | | MAIN AGENT PARTS BY MASS | CURING AGENT PARTS BY MASS | MAIN AGENT PARTS BY MASS | CURING AGENT PARTS BY MASS | | | | | | | | | | | | |
|---|---|---|---|---|---|---|---|---|---|---|---|---|---|---|---|---|---|---|---|---|---|---|---|
| EXAMPLE 1 | PVDC | 21 | 220 | 200 | 120 | 100 | 40 | 100 | 1.5 | — | — | 3.5 | 0.06 | NO | 1.7 | 38/34 | ○ | ○ | 5 | 500 | 300 | 200 | 0.5 |
| EXAMPLE 2 | PVDC | 21 | 220 | 200 | 120 | 100 | 40 | 100 | 1.5 | — | — | 16.5 | 0.25 | YES | 1.5 | 38/34 | ○ | ○ | 15 | 500 | 310 | 290 | 1.8 |
| EXAMPLE 3 | PVDC | 21 | 220 | 200 | 120 | 100 | 40 | 100 | 1.5 | — | — | 12.3 | 0.18 | YES | 1.6 | 38/34 | ○ | ○ | 9 | 500 | 300 | 270 | 1.4 |
| EXAMPLE 4 | PVDC | 21 | 220 | 200 | 120 | 100 | 40 | 100 | 2 | — | — | 11.5 | 0.08 | NO | 1.6 | 38/34 | ○ | ○ | 11 | 500 | 290 | 250 | 0.7 |
| EXAMPLE 5 | PVDC | 21 | 220 | 200 | 120 | 100 | 40 | 100 | 6 | — | — | 13.6 | 0.05 | NO | 1.5 | 38/34 | ○ | ○ | 21 | 500 | 300 | 190 | 0.5 |
| EXAMPLE 6 | PVDC | 12 | 210 | 200 | 80 | 70 | 40 | 100 | 1.5 | — | — | 19.8 | 0.29 | YES | 1.4 | 37/33 | ○ | ○ | 4 | 298 | 310 | 350 | 2.6 |
| EXAMPLE 7 | PVDC | 12 | 210 | 200 | 80 | 70 | 40 | 100 | 1.5 | — | — | 9 | 0.15 | YES | 1.5 | 37/33 | ○ | ○ | 2 | 298 | 300 | 260 | 1.2 |
| EXAMPLE 8 | PVDC | 41 | 220 | 200 | 120 | 100 | 40 | 100 | 2 | — | — | 11 | 0.07 | NO | 1.6 | 38/34 | ○ | ○ | 19 | 500 | 290 | 220 | 0.6 |
| EXAMPLE 9 | PE (CROSSLINKED) | 11 | 400 | 250 | 140 | 150 | 31 | 100 | 1.5 | — | — | 4 | 0.07 | NO | 0.9 | 60/60 | ○ | ○ | 4 | 500 | 300 | 220 | 0.6 |
| EXAMPLE 10 | PE | 11 | 330 | 250 | 190 | 200 | 31 | 100 | 1.5 | — | — | 4 | 0.08 | NO | 0.9 | UNMEASURABLE | △ | △ | 4 | 500 | 300 | 230 | 0.7 |
| EXAMPLE 11 | PVDC | 21 | 220 | 200 | 120 | 100 | 40 | — | — | 100 | 5 | 15 | 0.05 | YES | 1.7 | 38/34 | ○ | ○ | 6 | 500 | 300 | 50 | 0.5 |
| EXAMPLE 12 | PVDC | 21 | 220 | 200 | 120 | 100 | 40 | — | — | 100 | 6 | 20 | 0.3 | YES | 1.7 | 38/34 | ○ | ○ | 6 | 500 | 300 | 60 | 3.2 |
| COMPARATIVE EXAMPLE 1 | PVDC | 6 | 220 | 200 | 55 | 50 | 40 | 100 | 6 | — | — | 14 | 0.05 | NO | 1.5 | 40/41 | △ | ○ | 3 | 500 | 200 | 230 | 0.5 |
| COMPARATIVE EXAMPLE 2 | PE | 10 | 230 | 220 | 350 | 600 | 31 | 100 | 1.5 | — | — | 4 | 0.07 | NO | 0.9 | UNMEASURABLE | × | × | 5 | 500 | 200 | 220 | 0.7 |
| COMPARATIVE EXAMPLE 3 | Ny | 25 | 900 | 900 | 100 | 100 | 46 | 100 | 1.5 | — | — | 25 | 0.2 | YES | 1.1 | 1/2 | × | △ | 10 | 500 | 210 | 310 | 1.6 |
| COMPARATIVE EXAMPLE 4 | SILICONE | 100 | 25 | 30 | 400 | 500 | 16 | — | — | — | — | — | — | — | — | 1/1 | × | △ | 40 | — | — | — | — |

## INTERNATIONAL SEARCH REPORT

| International application No. |
| --- |
| PCT/JP2017/025199 |

A. CLASSIFICATION OF SUBJECT MATTER
*B65D5/72(2006.01)i*

According to International Patent Classification (IPC) or to both national classification and IPC

B. FIELDS SEARCHED

Minimum documentation searched (classification system followed by classification symbols)
B65D5/72, B65D25/52, B65D83/08, A61B5/00

Documentation searched other than minimum documentation to the extent that such documents are included in the fields searched
| | | | |
| --- | --- | --- | --- |
| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | Microfilm of the specification and drawings annexed to the request of Japanese Utility Model Application No. 168338/1987(Laid-open No. 73125/1989) (Wataru HIROSHIMA), 17 May 1989 (17.05.1989), page 3, line 9 to page 4, line 5; fig. 1 to 2 (Family: none) | 1-11,21-22 |
| Y | JP 2008-265839 A (Kureha Corp.), 06 November 2008 (06.11.2008), paragraphs [0021] to [0023] (Family: none) | 1-11,22 |

| ☒ Further documents are listed in the continuation of Box C. | ☐ See patent family annex. |
| --- | --- |

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 05 September 2017 (05.09.17) | 19 September 2017 (19.09.17) |

| Name and mailing address of the ISA/ | Authorized officer |
| --- | --- |
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

International application No.

PCT/JP2017/025199

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 8-19522 A  (Toshiba Corp.),<br>23 January 1996 (23.01.1996),<br>paragraphs [0008], [0025] to [0051]; fig. 1 to 5<br>(Family: none) | 1-22 |
| Y | JP 2015-199526 A  (Nippon Paper Crecia Co., Ltd.),<br>12 November 2015 (12.11.2015),<br>paragraphs [0010] to [0016]; fig. 2<br>(Family: none) | 5-11 |
| Y | Microfilm of the specification and drawings annexed to the request of Japanese Utility Model Application No. 160934/1987(Laid-open No. 66323/1989)<br>(Nobuhiko SATO),<br>27 April 1989 (27.04.1989),<br>page 3, line 15 to page 5, line 3; fig. 1 to 3<br>(Family: none) | 6-11 |
| Y | JP 6-508088 A  (Dow Italia S.p.A.),<br>14 September 1994 (14.09.1994),<br>page 2, lower right column to page 4, upper left column; fig. 1 to 3<br>& WO 1992/022479 A1<br>pages 5 to 9; fig. 1 to 3<br>& EP 0518347 A1 | 10-11 |
| Y | JP 7-32500 A  (Toyobo Co., Ltd.),<br>03 February 1995 (03.02.1995),<br>paragraphs [0002], [0005] to [0006], [0027] to [0028]<br>(Family: none) | 12-22 |
| Y | JP 2016-39846 A  (Osamu MOTOYAMA),<br>24 March 2016 (24.03.2016),<br>paragraphs [0017], [0027], [0043]<br>(Family: none) | 16-22 |
| Y | JP 9-316411 A  (Sekisui Chemical Co., Ltd.),<br>09 December 1997 (09.12.1997),<br>paragraph [0035]<br>(Family: none) | 18-22 |
| P,X | JP 2017-111787 A  (Asahi Kasei Corp.),<br>22 June 2017 (22.06.2017),<br>paragraphs [0012] to [0057]<br>(Family: none) | 12-22 |

**INTERNATIONAL SEARCH REPORT**

International application No.

PCT/JP2017/025199

C (Continuation).    DOCUMENTS CONSIDERED TO BE RELEVANT

| Category* | Citation of document, with indication, where appropriate, of the relevant passages | Relevant to claim No. |
|---|---|---|
| A | WO 2013/038522 A1  (Asahi Kasei Chemicals Corp.), 21 March 2013 (21.03.2013), & US 2014/0263527 A1    & EP 2757048 A1 & CN 103732505 A | 1-22 |
| A | JP 2009-1340 A  (Yorio KISHINO), 08 January 2009 (08.01.2009), (Family: none) | 1-22 |

Form PCT/ISA/210 (continuation of second sheet) (January 2015)

<table>
<tr><td>**INTERNATIONAL SEARCH REPORT**</td><td>International application No.<br>PCT/JP2017/025199</td></tr>
</table>

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
        See extra sheet.

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**       ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (January 2015)

**INTERNATIONAL SEARCH REPORT**

| International application No. |
| --- |
| PCT/JP2017/025199 |

<u>Continuation of Box No.III of continuation of first sheet(2)</u>

Document: JP 2015-83484 A (Asahi Kasei Chemicals Corp.) 30 April 2015 (30.04.2015), paragraphs [0014] to [0019]; fig. 1 to 4 (Family: none)
Claims are classified into the following two inventions.
(Invention 1) claims 1-11
In view of the matter disclosed in the document shown above, it is considered that claims 1-11 have a special technical feature which is "a box for housing a pressure-sensitive-adhesive film therein ······ the front panel has a smaller height than the back panel and side panels, the height being 50-90% of the outer diameter of the core tube of the roll". Claims 1-11 are hence classified as invention 1.
(Invention 2) claims 12-22
There is no same or corresponding special technical feature between claims 12-22 and claim 1.
Further, claims 12-22 are not dependent on claim 1, and have no relationship such that these claims are substantially same as or equivalent to any claim classified into Invention 1.
Consequently, claims 12-22 cannot be classified into Invention 1.
Claims 12-22 have a special technical feature which is "an infection-preventing film that includes a base film in which the flow-direction (machine-direction) modulus at 2% strain and the width-direction (transverse-direction) modulus at 2% strain satisfy 190-600 MPa each and the flow-direction (MD) and width-direction (TD) tensile elongations satisfy 60-200% each", and are hence classified as invention 2.

Form PCT/ISA/210 (extra sheet) (January 2015)

**EP 3 486 195 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016137602 A
- JP 2017089940 A
- JP 2017120231 A
- JP 2015083484 A